# EUROPEAN PATENT APPLICATION

(11) **EP 3 527 657 A1**
(43) Date of publication of application: **21.08.2019**
(21) Application number: 17863159.4
(22) Date of filing: 17.10.2017
(51) Int. Cl.: C12N 5/071, A61K 31/198, A61K 31/336, A61K 31/365, A61K 31/4458, A61K 31/7004, A61K 35/12, A61K 35/34, A61K 45/00, A61K 45/06, A61P 43/00, C12N 5/10, C12N 9/99

(54) **UNDIFFERENTIATED STEM CELL-REMOVING AGENT, AND METHOD FOR REMOVING UNDIFFERENTIATED STEM CELLS**

(30) Priority: 17.10.2016 JP 2016203839
(71) Applicant: Keio University, Tokyo 108-8345 (JP)
(72) Inventor: TOHYAMA, Shugo, Tokyo 160-8582 (JP); FUKUDA, Keiichi, Tokyo 160-8582 (JP); FUJITA, Jun, Tokyo 160-8582 (JP); TANOSAKI, Sho, Tokyo 160-8582 (JP)
(74) Representative: HGF Limited
(86) International application number: PCT/JP2017/037495
(87) International publication number: WO 2018/074457

(57) **Abstract**

An undifferentiated stem cell-removing agent which contains at least one kind selected from the group consisting of a fatty acid synthesis inhibitor, a fatty acid utilization inhibitor, and a cholesterol synthesis inhibitor; a method for removing undifferentiated stem cells which includes culturing a cell mixture that contains an undifferentiated stem cell and a differentiated cell in the presence of the undifferentiated stem cell-removing agent; and a production method of cells for transplantation which includes the following steps (i) and (ii): a step (i) of inducing a desired differentiated cell from an undifferentiated stem cell, and a step (ii) of culturing the cell mixture obtained in the step (i) in the presence of the undifferentiated stem cell-removing agent are provided.

## Description

### Technical Field

The present invention relates to an undifferentiated stem cell-removing agent, and a method for removing undifferentiated stem cells. The present invention further relates to a culture medium containing the undifferentiated stem cell-removing agent, a method for producing cells for transplantation, and a pharmaceutical composition.

Priority is claimed on Japanese Patent Application No. 2016-203839, filed October 17, 2016, the content of which is incorporated herein by reference.

### Background Art

Research on pluripotent stem cells such as embryonic stem cells (ES cells) and induced pluripotent stem cells (iPS cells) has progressed in recent years. Because these cells are pluripotent, it is becoming possible to produce cells that differentiate into a desired lineage through differentiation and induction, and to use the produced cells in medical transplantation.

Differentiation and induction of embryonic stem cells and induced pluripotent stem cells are generally carried out in vitro.

However, in in vitro, it is difficult to give rise to the differentiation of a target lineage with respect to all stem cells. Therefore undifferentiated stem cells may remain partly after the differentiation and induction manipulation. Because such undifferentiated stem cells have proliferative activity and can differentiate into various kinds of cells, in a case where these undifferentiated stem cells are transplanted into a living body, a teratoid tumor may be formed (refer to, for example, NPL 1). For this reason, it is difficult to directly transplant a cell population which is produced through differentiation and induction of stem cells into a living body as it is so as to be used in treatment.

Accordingly, it is necessary to remove undifferentiated stem cells in order to safely carry out transplantation of cells differentiated and induced from stem cells into a living body so as to obtain ideal therapeutic effects. In addition, in a case where transplantation is carried out with undifferentiated stem cells remaining, it is necessary to suppress proliferation of undifferentiated stem cells in a living body.

A method in which undifferentiated stem cells can be selectively removed in culture conditions has been reported to date (refer to, for example, NPLs 2 to 4). A research group of the inventors of the present invention has also developed a method for selecting cardiomyocytes from non-cardiomyocytes and undifferentiated stem cells (refer to PTLs 1 to 6 and NPL 5).

### Citation List

### Patent Literature

[PTL 1] PCT International Publication No. WO2006/022377
[PTL 2] PCT International Publication No. WO2007/088874
[PTL 3] PCT International Publication No. WO2009/017254
[PTL 4] PCT International Publication No. WO2010/114136
[PTL 5] PCT International Publication No. WO2011/052801
[PTL 6] PCT International Publication No. WO2016/010165

### Non-Patent Literature

[NPL 1] Miura et al., (2009) Nature Biotech., 8, 743-745.
[NPL 2] Ben-David, et al., (2013) Cell Stem Cell, 12, 167-179.
[NPL 3] Wang, et al., (2009) Science, 325, 435-439.
[NPL 4] Shiraki, et al., (2014) Cell Metabolism, 19, 780-794.
[NPL 5] Tohyama, et al., (2016) Cell Metabolism, 23, 663-674.

### Summary of Invention

### Technical Problem

However, there is still room for further study on a technique of removing undifferentiated stem cells for the purpose of reducing a residual ratio of undifferentiated stem cells.

The present invention has been made in view of the above circumstances, and an object of the present invention is to provide an undifferentiated stem cell-removing agent which is capable of removing undifferentiated stem cells with high efficiency, and a method for removing undifferentiated stem cells.

### Solution to Problem

As a result of intensive studies to achieve the above object, the inventors of the present invention have found that cell death of undifferentiated stem cells can be induced by culturing undifferentiated stem cells in the presence of a fatty acid synthesis inhibitor, a fatty acid utilization inhibitor, or a cholesterol synthesis inhibitor. Therefore the inventors of the present invention have completed the present invention.

That is, the present invention includes the following aspects.
[1] An undifferentiated stem cell-removing agent, containing at least one kind selected from the group consisting of a fatty acid synthesis inhibitor, a fatty acid utilization inhibitor, and a cholesterol synthesis inhibitor.
[2] The undifferentiated stem cell-removing agent according to [1],
   in which the fatty acid synthesis inhibitor inhibits fatty acid synthesis by targeting at least one factor selected from the group consisting of an ATP citrate lyase, a fatty acid synthase, an acetyl-CoA carboxylase, and a malonyl-CoA decarboxylase, and
   the fatty acid utilization inhibitor inhibits fatty acid utilization by targeting a carnitine palmitoyltransferase 1.
[3] The undifferentiated stem cell-removing agent according to [1] or [2], in which a concentration of the fatty acid synthesis inhibitor or the fatty acid utilization inhibitor in an application subject is 0.1 to 500 µM.
[4] The undifferentiated stem cell-removing agent according to [1], in which the cholesterol synthesis inhibitor inhibits cholesterol synthesis by targeting at least one factor selected from the group consisting of an acetyl-CoA acetyltransferase, an HMG-CoA synthase, and an HMG-CoA reductase.
[5] The undifferentiated stem cell-removing agent according to [1] or [4], in which a concentration of the cholesterol synthesis inhibitor in an application subject is 0.01 to 50 µM.
[6] The undifferentiated stem cell-removing agent according to any one of [1] to [5], further containing at least one compound selected from the group consisting of glucose, glutamine, and methionine.
[7] An undifferentiated stem cell-removing agent, containing one or two or more kinds selected from the group consisting of Orlistat, C75, LY294002, SB204990, etomoxir, perhexiline, and simvastatin, and salts thereof.
[8] A culture medium containing the undifferentiated stem cell-removing agent according to any one of [1] to [7].
[9] A method for removing undifferentiated stem cells, including culturing a cell mixture that contains an undifferentiated stem cell and a differentiated cell in the presence of the undifferentiated stem cell-removing agent according to any one of [1] to [7].
[10] The method for removing undifferentiated stem cells according to [9], in which the undifferentiated stem cell is an induced pluripotent stem cell.
[11] The method for removing undifferentiated stem cells according to [9] or [10], in which the differentiated cell is a cardiomyocyte.
[12] A production method of cells for transplantation, including the following steps (i) and (ii):
   a step (i) of inducing a desired differentiated cell from an undifferentiated stem cell; and
   a step (ii) of culturing a cell mixture obtained in the step (i) in the presence of the undifferentiated stem cell-removing agent according to any one of [1] to [7].
[13] The production method according to [12], in which the undifferentiated stem cell is an induced pluripotent stem cell.
[14] The production method according to [12] or [13], in which the differentiated cell is a cardiomyocyte.
[15] A pharmaceutical composition for treating or preventing a disease caused by proliferation of an undifferentiated stem cell, in a subject into which a differentiated cell induced from the undifferentiated stem cell is transplanted, the pharmaceutical composition including the undifferentiated stem cell-removing agent according to any one of [1] to [7].
[16] The pharmaceutical composition according to [15], in which the undifferentiated stem cell is an induced pluripotent stem cell.
[17] The pharmaceutical composition according to [15] or [16], in which the differentiated cell induced from the undifferentiated stem cell is a cardiomyocyte.

### Advantageous Effects of Invention

According to the undifferentiated stem cell-removing agent of the present invention, it is possible to highly efficiently remove undifferentiated stem cells.

According to the method for removing undifferentiated stem cells of the present invention, it is possible to highly efficiently remove undifferentiated stem cells.

### Brief Description of Drawings

FIG. 1 is a diagram explaining an outline of a fatty acid synthetic pathway in a cell.
FIG. 2 shows images showing results of comparison of FASN expression between cardiomyocytes derived from human iPS cells and iPS cells in Experimental Example 1.
FIG. 3 shows images of undifferentiated stem cell lines cultured in Experimental Example 2.
FIG. 4 shows images of undifferentiated stem cell lines cultured in Experimental Example 3.
FIG. 5 shows images of purified and refined cardiomyocytes cultured in Experimental Example 4.
FIG. 6 shows images of fibroblasts cultured in Experimental Example 5.
FIG. 7 shows images of a cell mixture containing cardiomyocytes derived from human iPS cells and human iPS cells cultured in Experimental Example 6.
FIG. 8 is a graph showing results of comparing the number of Oct4-positive colonies in a cell mixture cultured in a culture medium containing a fatty acid synthesis inhibitor and a culture medium not containing the fatty acid synthesis inhibitor in Experimental Example 6.
FIG. 9 is a diagram explaining an outline of a cholesterol synthetic pathway in a cell.
FIG. 10 shows images showing results of comparison of FASN expression between cardiomyocytes derived from human iPS cells and human iPS cells in Experimental Example 7.
FIG. 11 is a photograph of Western blotting, which shows results of comparison of FASN expression between cardiomyocytes derived from human iPS cells and human iPS cells in Experimental Example 7.
FIG. 12 shows an image of an undifferentiated stem cell line cultured in Experimental Example 8.
FIG. 13 shows images showing wells of human iPS cells cultured in a culture medium (PA-BSA) containing the fatty acid synthesis inhibitor and fatty acids and a culture medium (BSA) containing the fatty acid synthesis inhibitor and not containing fatty acids in Experimental Example 9.
FIG. 14 is a graph showing results of comparing the number of viable cells in human iPS cultured in the culture medium (PA-BSA) containing the fatty acid synthesis inhibitor and fatty acids and the culture medium (BSA) containing the fatty acid synthesis inhibitor and not containing fatty acids in Experimental Example 9.
FIG. 15 is a photograph of Western blotting, which shows results of comparing the expression of FASN between an undifferentiated stem cell line into which FASN siRNA is introduced and an undifferentiated stem cell line into which negative control siRNA (N/C siRNA) is introduced in Experimental Example 10.
FIG. 16 is a graph showing results of comparison of cell proliferation between an undifferentiated stem cell line (FASN ND) into which FASN siRNA is introduced and an undifferentiated stem cell line (N/C) into which negative control siRNA is introduced in Experimental Example 10.
FIG. 17 is a graph showing results of comparison of cell proliferation between an undifferentiated stem cell line (FASN KD) into which FASN siRNA is introduced and an undifferentiated stem cell line (N/C) into which negative control siRNA is introduced, which are cultured in the culture medium (PA-BSA) containing the fatty acid synthesis inhibitor and fatty acids and the culture medium (BSA) containing the fatty acid synthesis inhibitor and not containing fatty acids in Experimental Example 11.

### Description of Embodiments

### <<Definition and the like>>

In the present specification, the term "undifferentiated stem cell" is used as a concept encompassing pluripotent stem cells having differentiation pluripotency, and may include embryonic stem cells (ES cells), induced pluripotent stem cells (iPS cells), cells having differentiation pluripotency induced from these stem cells, and various somatic stem cells. The term "undifferentiated stem cell" is not particularly limited as long as it is a cell having differentiation pluripotency, and also includes unknown cells having properties equivalent to those of the ES cells and iPS cells exemplified above.

It can be determined whether the cells are undifferentiated stem cells based on the presence or absence of properties specific to undifferentiated stem cells, and the expression of various markers specific to undifferentiated stem cells. For example, examples of properties specific to undifferentiated stem cells include a property that has the ability to self-replicate and can differentiate into a different kind of cell having a property different from that of undifferentiated stem cells. In addition, examples thereof further include teratoma-forming ability, chimeric mouse-forming ability, and the like as properties specific to undifferentiated stem cells.

Various markers specific to undifferentiated stem cells (hereinafter referred to as an "undifferentiated stem cell marker") are a factor that is specifically expressed in undifferentiated stem cells, and examples thereof include Oct3/4, Nanog, Sox2, SSEA-1, SSEA-3, SSEA-4, TRA1-60, TRA1-81, Lin28, Fbx15, and the like. In a case where expression of at least one of these undifferentiated stem cell markers is observed, it can be determined that this cell is an undifferentiated stem cell. One kind of the undifferentiated stem cell marker may be used alone, or two or more kinds thereof may be used in combination. In one embodiment, cells expressing Oct3/4 may be determined as undifferentiated stem cells. The expression of the undifferentiated stem cell marker in cells can be checked using known methods such as RT-PCR and microarray.

The undifferentiated stem cell may be a mammalian undifferentiated stem cell, a rodent undifferentiated stem cell, a primate undifferentiated stem cell, or a human undifferentiated stem cell. Examples thereof include human-derived undifferentiated stem cells, and more specific examples thereof include human iPS cells, human ES cells, and the like.

In the present specification, the term "differentiated cell" refers to a cell that does not have the properties of the "undifferentiated stem cells." Differentiated cells may be cells induced and differentiated from undifferentiated stem cells, but do not have differentiation pluripotency. The differentiated cells may be, for example, cells differentiated from ES cells, or cells differentiated from iPS cells. Examples of differentiated cells include cardiomyocytes; muscle cells; fibroblasts; nerve cells; immune cells such as lymphocytes; vascular cells; ocular cells such as retinal pigment epithelial cells; blood cells such as megakaryocytes and erythrocytes; and other tissue cells; and precursor cells thereof.

### <<Undifferentiated Stem Cell-Removing Agent>>

In one embodiment, the present invention provides an undifferentiated stem cell-removing agent.

The undifferentiated stem cell-removing agent of the present embodiment contains at least one kind selected from the group consisting of a fatty acid synthesis inhibitor, a fatty acid utilization inhibitor, and a cholesterol inhibitor.

Hereinafter, the present invention will be explained based on embodiments.

As shown in Examples to be described later, the inventors of the present invention have found that cell death can be induced when the fatty acid synthesis inhibitor or a fatty acid degradation inhibitor (fatty acid utilization inhibitor) is brought into contact with undifferentiated stem cells. The inventors of the present invention have found that the reason for this is because a fatty acid synthetic pathway is enhanced in human iPS cells (undifferentiated stem cells). Presumably, the fatty acid synthetic pathway and a metabolic pathway thereof are perceived to be very important for the survival of undifferentiated stem cells. For this reason, it is perceived that cell death is induced in undifferentiated stem cells by inhibition of related fatty acid synthesis or fatty acid degradation.

Furthermore, the inventors of the present invention have found that cell death can be induced by bringing a cholesterol synthesis inhibitor into contact with undifferentiated stem cells. Cholesterol is a component of a cell membrane, and the cholesterol synthetic pathway is perceived to be very important for the survival of undifferentiated stem cells. For this reason, it is perceived that cell death is induced in undifferentiated stem cells by inhibition of related cholesterol synthesis.

In addition, even in a case where a cell population in which undifferentiated stem cells remain is transplanted into a living body, it is possible to selectively remove undifferentiated stem cells in the living body by administering the fatty acid synthesis inhibitor, the fatty acid utilization inhibitor, or the cholesterol synthesis inhibitor to the living body.

In the present specification, the "fatty acid utilization inhibitor" is an inhibitor having a function of inhibiting a reaction of a metabolic pathway involved in the synthesis of a substance utilizing fatty acids, and/or a function of inhibiting a reaction of a metabolic pathway involved in the degradation of fatty acids. In the present specification, the "fatty acid utilization inhibitor" includes the "fatty acid degradation inhibitor" that inhibits the reaction of the metabolic pathway involved in the degradation of fatty acids.

As the metabolic pathway involved in the degradation of fatty acids, a metabolic pathway involving β-oxidation is preferred. The fatty acid utilization inhibitor is preferably a "fatty acid metabolism inhibitor involved in β-oxidation." Examples of fatty acid metabolism related to β-oxidation include β-oxidation, synthesis of fatty acid metabolites used for β-oxidation, incorporation of fatty acid metabolites used for β-oxidation into mitochondria, and the like.

In addition, as a metabolic pathway involved in the synthesis of a substance utilizing fatty acids, metabolic pathways involved in the synthesis of triglycerides and/or phospholipids are preferred. It is preferable that the fatty acid utilization inhibitor be a "fatty acid metabolism inhibitor involved in the synthesis of triglycerides and/or phospholipids."

The type of these inhibitors is not particularly limited, and any substance may be used as long as it has a function of inhibiting fatty acid synthesis, inhibiting fatty acid utilization, or inhibiting cholesterol synthesis, and may be an organic substance or an inorganic substance. The organic substance is preferably an organic compound, and examples thereof include a low-molecular-weight compound, a nucleic acid, a peptide, a protein, and the like.

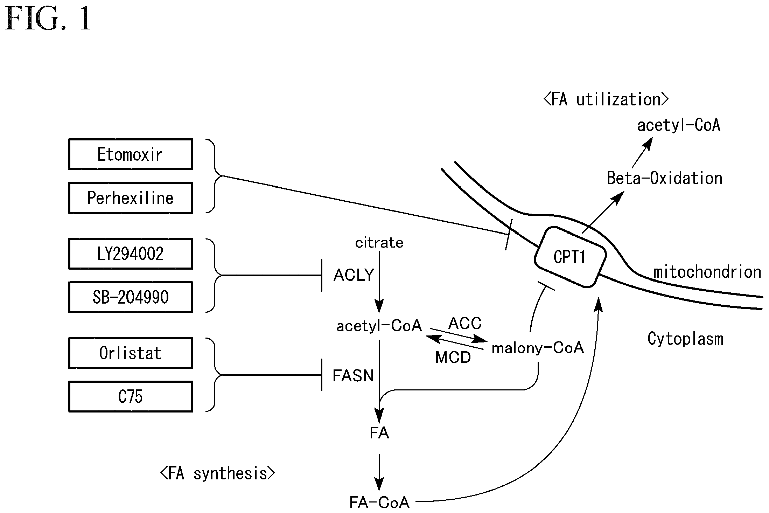

FIG. 1 is a diagram illustrating an outline of a fatty acid synthetic pathway and a fatty acid degradation (fatty acid utilization) pathway in the cytoplasm of natural cells. Acetyl-CoA is synthesized from citrate by ATP citrate lyase (ACLY), and a fatty acid (FA) is synthesized from acetyl-CoA and malonyl-CoA by fatty acid synthase (FASN).

Acetyl-CoA carboxylase (ACC) catalyzes the generation of malonyl-CoA from acetyl-CoA. Malonyl-CoA decarboxylase (MCD) catalyzes the generation of acetyl-CoA from malonyl-CoA.

The synthesized fatty acid becomes fatty acid acyl-CoA (FA-CoA) by the action of acyl-CoA synthetase (ACS), undergoes several reactions, and consequently, the resultant product is transported into mitochondria by carnitine palmitoyltransferase-1 (CPT1), and then used in β-oxidation.

The fatty acid synthesis inhibitor of the present embodiment may inhibit fatty acid synthesis by completely or partially inhibiting any of reactions of the fatty acid synthetic pathway as shown in FIG. 1. The fatty acid synthesis inhibitor of the present embodiment may inhibit fatty acid synthesis by targeting at least one factor selected from the group consisting of an ATP citrate lyase, a fatty acid synthase, an acetyl-CoA carboxylase, and a malonyl-CoA decarboxylase. Preferably, the fatty acid synthesis inhibitor of the present embodiment may inhibit fatty acid synthesis by targeting at least one factor selected from the group consisting of the ATP citrate lyase, the fatty acid synthase, and the acetyl-CoA carboxylase, and may inhibit fatty acid synthesis by targeting at least one factor selected from the group consisting of the ATP citrate lyase and the fatty acid synthase.

The fatty acid degradation inhibitor of the present embodiment may inhibit fatty acid degradation by completely or partially inhibiting any of reactions of the fatty acid degradation pathways involved in β-oxidation as shown in FIG. 1. The fatty acid degradation inhibitor of the present embodiment may inhibit fatty acid degradation by targeting the carnitine palmitoyltransferase 1.

In addition to being used for β-oxidation, fatty acids are also used for the synthesis of triglycerides and phospholipids. The fatty acid acyl-CoA is used for the synthesis of lysophosphatidic acid (LPA) by glycerol-3-phosphate acyltransferase (GPAT). In addition, the fatty acid acyl-CoA is used for the synthesis of phosphatidic acid (PA) by acyl-glycerol-3-phosphate acyltransferase (AGPAT). Furthermore, the fatty acid acyl-CoA is used for the synthesis of diacylglycerol (DAG, DG) by phosphatidate phosphatase (PAP). Furthermore, the fatty acid acyl-CoA is used for the synthesis of triglycerides by diacylglycerol acyltransferase (DGAT). Furthermore, the fatty acid acyl-CoA is also degraded to fatty acids and glycerol by monoacylglycerol lipase (MAGL). The fatty acid utilization inhibitor of the present embodiment may inhibit fatty acid utilization by completely or partially inhibiting any of reactions of these pathways utilizing fatty acids.

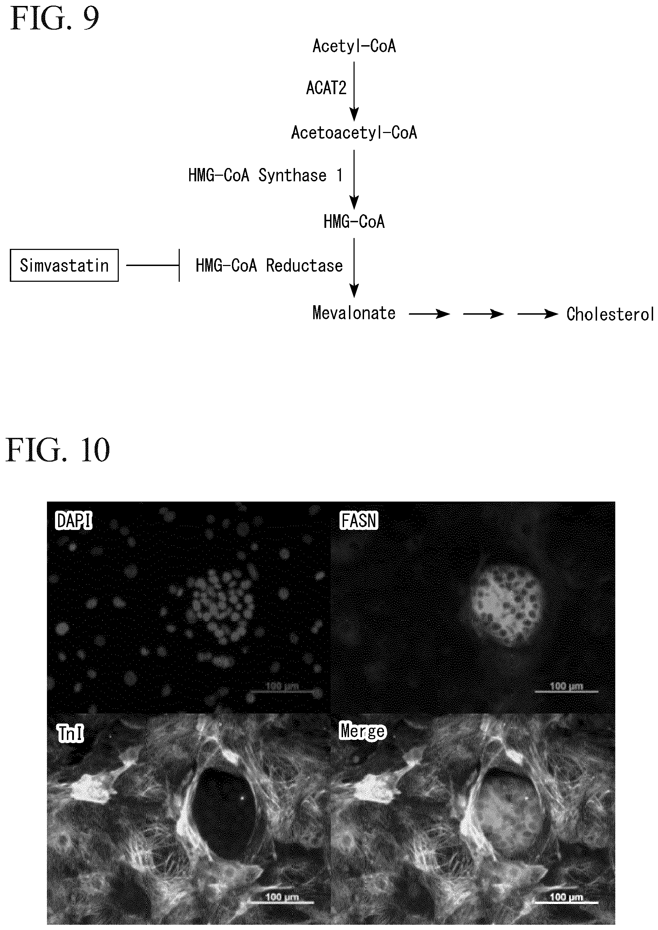

FIG. 9 is a diagram explaining an outline of a cholesterol synthetic pathway. Acetoacetyl-CoA is synthesized from acetyl-CoA by acetyl-CoA acetyltransferase (ACAT2). Hydroxymethylglutaryl-CoA (HMG-CoA) is synthesized from acetoacetyl-CoA by hydroxymethylglutaryl-CoA Syntase 1 (HMG-CoA Syntase 1). Mevalonate is synthesized from HMG-CoA by hydroxymethylglutaryl-CoA reductase (HMG-CoA reductase). And then, after several reactions, consequently cholesterol is synthesized.

The cholesterol synthesis inhibitor of the present embodiment may inhibit cholesterol synthesis by completely or partially inhibiting any of reactions in the cholesterol synthetic pathway as shown in FIG. 9. The cholesterol synthesis inhibitor of the present embodiment may inhibit cholesterol synthesis by targeting at least one factor selected from the group consisting of an acetyl-CoA acetyltransferase, an HMG-CoA synthase, and an HMG-CoA reductase. Preferably, the cholesterol synthesis inhibitor of the present embodiment may inhibit cholesterol synthesis by targeting HMG-CoA reductase.

The fatty acid synthesis inhibitor may be used alone or in combination of two or more kinds thereof. The fatty acid utilization inhibitor may be used alone or in combination of two or more kinds thereof. The cholesterol synthesis inhibitor may be used alone or in combination of two or more kinds thereof.

The undifferentiated stem cell-removing agent may contain one fatty acid synthesis inhibitor, or two or more fatty acid synthesis inhibitors in combination. The undifferentiated stem cell-removing agent may contain one fatty acid utilization inhibitor, or two or more fatty acid utilization inhibitors in combination. The undifferentiated stem cell-removing agent may contain one or two or more cholesterol synthesis inhibitors in combination. The undifferentiated stem cell-removing agent can contain one kind or two or more kinds in combination selected from the group consisting of the fatty acid synthesis inhibitor, the fatty acid utilization inhibitor, and the cholesterol synthesis inhibitor.

The fatty acid synthesis inhibitor of the present embodiment may inhibit fatty acid synthesis by targeting the fatty acid synthase. Examples of the fatty acid synthesis inhibitor that inhibits fatty acid synthesis by targeting the fatty acid synthase include Orlistat, C75, flavonoise, Epigallocatechin-3-gallate (EGCG), and the like, and Orlistat and C75 are preferable.

As Orlistat and C75, commercially available products can be used. In addition, as long as the fatty acid synthesis inhibitor of the present embodiment has the same function as Orlistat or C75, the fatty acid synthesis inhibitor may be a salt or a derivative of these compounds.

Orlistat is known as a compound represented by Formula (1), (N-formyl-L-leucine-(1S)-1-[[(2S,3S)-3-hexyl-4-oxo-2-oxetanyl]methyl]dodecyl ester).

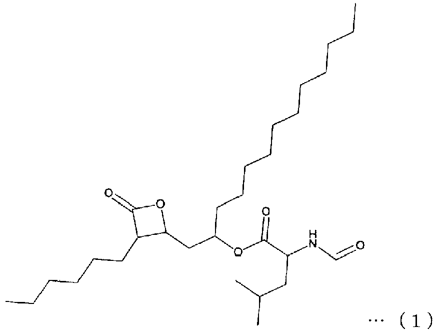

C75 is known as a compound represented by Formula (2), (tetrahydro-4-methylene-2R-octyl-5-oxo-3S-furancarboxylic acid).

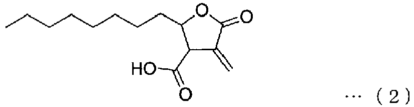

The fatty acid synthesis inhibitor according to the present embodiment may inhibit fatty acid synthesis by targeting the ATP citrate lyase. Preferred examples of the fatty acid synthesis inhibitor that inhibits fatty acid synthesis by targeting the ATP citrate lyase include LY294002 and SB204990. As LY294002 and SB204990, commercially available products can be used. In addition, as long as the fatty acid synthesis inhibitor of the present embodiment has the same function as LY294002 or SB204990, the fatty acid synthesis inhibitor may be a salt or a derivative of these compounds.

The fatty acid degradation inhibitor of the present embodiment may inhibit fatty acid degradation by targeting the carnitine palmitoyltransferase 1. Examples of the fatty acid degradation inhibitor that inhibits fatty acid degradation by targeting the carnitine palmitoyltransferase 1 include Etomoxir, Perhexiline, Ranolazine, and the like, and etomoxir and perhexiline are preferred. As etomoxir and perhexiline, commercially available products can be used. In addition, as long as the fatty acid synthesis inhibitor of the present embodiment has the same function as etomoxir or perhexiline, the fatty acid synthesis inhibitor may be a salt or a derivative of these compounds.

The fatty acid synthesis inhibitor of the present embodiment may inhibit fatty acid synthesis by targeting the acetyl-CoA carboxylase. Examples of the fatty acid synthesis inhibitor that inhibits fatty acid synthesis by targeting the acetyl-CoA carboxylase include Soraphen A, TOFA, A769662, Metformin, AICAR, and the like, and TOFA and A769662 are preferred. As TOFA and A769662, commercially available products can be used. In addition, as long as the fatty acid synthesis inhibitor of the present embodiment has the same function as TOFA or A769662, the fatty acid synthesis inhibitor may be a salt or a derivative of these compounds.

The fatty acid synthesis inhibitor of the present embodiment may inhibit fatty acid synthesis by targeting the acyl CoA synthase. Examples of the fatty acid synthesis inhibitor that inhibits fatty acid synthesis by targeting the acyl-CoA synthetase include Triascin C, TZDs, and the like.

In addition, SREBP is known as a transcription factor that controls the expression of the ATP citrate lyase, acetyl-CoA carboxylase, fatty acid synthase, glycerol-3-phosphate acyltransferase, acyl CoA synthase, and the like. The fatty acid synthesis inhibitor and/or the fatty acid utilization inhibitor of the embodiment may inhibit the function of SREBP, and examples thereof include Fatostatin, FGH110019, and the like.

The cholesterol synthesis inhibitor of the present embodiment may inhibit cholesterol synthesis by targeting the HMG-CoA reductase. Examples of the cholesterol synthesis inhibitor that inhibits cholesterol synthesis by targeting the HMG-CoA reductase include Pravastatin, Simvastatin, Fluvastatin, Atorvastatin, Pitavastatin, Rosuvastatin, Cerivastatin, Lovastatin, Mevastatin, and the like. Pravastatin, Simvastatin, Fluvastatin, Atorvastatin, Pitavastatin, and Rosuvastatin are preferable, and Simvastatin is more preferable. As Pravastatin, Simvastatin, Fluvastatin, Atorvastatin, Pitavastatin, and Rosuvastatin, commercially available products can be used. In addition, as long as the cholesterol synthesis inhibitor of the present embodiment has the same function as Pravastatin, Simvastatin, Fluvastatin, Atorvastatin, Pitavastatin, or Rosuvastatin, the cholesterol synthesis inhibitor may be a salt or a derivative of these compounds.

The undifferentiated stem cell-removing agent of the embodiment may contain one or two or more kinds selected from the group consisting of Orlistat, C75, LY294002, SB204990, etomoxir, perhexiline, and simvastatin, and salts thereof.

In the undifferentiated stem cell-removing agent of the present embodiment, a concentration of the fatty acid synthesis inhibitor contained in an application subject may be 0.1 to 500 µM. In the present specification, the term "M" as a unit means mol/L. In addition, in the present specification, the term "in the application subject" means in a culture medium or in the blood.

Furthermore, in the undifferentiated stem cell-removing agent of the present embodiment, a concentration of the fatty acid utilization inhibitor contained in the application subject may be 0.1 to 500 µM.

Furthermore, in the undifferentiated stem cell-removing agent of the present embodiment, a concentration of the fatty acid synthesis inhibitor contained in the application subject may be 0.1 to 500 µg/mL.

Furthermore, in the undifferentiated stem cell-removing agent of the present embodiment, a concentration of the fatty acid utilization inhibitor contained in the application subject may be 0.1 to 500 µg/mL.

Furthermore, in the undifferentiated stem cell-removing agent of the present embodiment, a concentration of the cholesterol synthesis inhibitor contained in the application subject may be 0.01 to 50 µM.

Furthermore, in the undifferentiated stem cell-removing agent of the present embodiment, a concentration of the cholesterol synthesis inhibitor contained in the application subject may be 0.01 to 50 µg/mL.

In a case where the fatty acid synthesis inhibitor according to the present embodiment is Orlistat, a concentration of Orlistat in the application subject is preferably 0.1 to 500 µM, more preferably 1 to 50 µM, and even more preferably 3 to 15 µM.

In a case where the fatty acid synthesis inhibitor according to the present embodiment is C75, a concentration of C75 in the application subject is preferably 0.1 to 500 µg/mL, more preferably 1 to 100 µg/mL, and even more preferably 5 to 50 µg/mL.

In a case where the fatty acid synthesis inhibitor according to the present embodiment is SB204990, a concentration of SB204990 in the application subject is preferably 0.1 to 500 µM, more preferably 1 to 200 µM, and even more preferably 20 to 100 µM.

In a case where the fatty acid synthesis inhibitor according to the present embodiment is LY294002, a concentration of LY294002 in the application subject is preferably 0.1 to 500 µM, more preferably 1 to 200 µM, and even more preferably 20 to 100 µM.

In a case where the fatty acid degradation inhibitor (fatty acid utilization inhibitor) according to the present embodiment is etomoxir, a concentration of etomoxir in the application subject is preferably 0.1 to 500 µM, more preferably 1 to 200 µM, and even more preferably 20 to 100 µM.

In a case where the fatty acid degradation inhibitor (fatty acid utilization inhibitor) according to the present embodiment is perhexiline, a concentration of perhexiline in the application subject is preferably 0.1 to 500 µM, more preferably 1 to 100 µM, and even more preferably 5 to 50 µM.

In a case where the cholesterol synthesis inhibitor according to the present embodiment is simvastatin, a concentration of simvastatin in the application subject is preferably 0.01 to 50 µM, more preferably 0.1 to 30 µM, and even more preferably 0.1 to 10 µM.

In a case where the fatty acid synthesis inhibitor according to the present embodiment is brought into contact with undifferentiated stem cells, the fatty acid synthesis inhibitor is incorporated into the cytoplasm of undifferentiated stem cells, and thereby completely or partially inhibiting any of reactions in the fatty acid synthetic pathway shown in FIG. 1.

In a case where the fatty acid degradation inhibitor according to the present embodiment is brought into contact with undifferentiated stem cells, the fatty acid degradation inhibitor is incorporated into the cytoplasm and/or mitochondria of undifferentiated stem cells, and thereby completely or partially inhibiting any of reactions in the fatty acid degradation pathway shown in FIG. 1.

In a case where the cholesterol synthesis inhibitor according to the present embodiment is brought into contact with undifferentiated stem cells, the cholesterol synthesis inhibitor is incorporated into the cytoplasm of undifferentiated stem cells, and thereby completely or partially inhibiting any of reactions in the cholesterol synthetic pathway shown in FIG. 9.

As long as the undifferentiated stem cell-removing agent of the present embodiment contains at least one kind selected from the group consisting of the fatty acid synthesis inhibitor, the fatty acid utilization inhibitor, and the cholesterol synthesis inhibitor, any undifferentiated stem cell-removing agent may be used. The undifferentiated stem cell-removing agent of the present embodiment may be any undifferentiated stem cell-removing agent as long as the undifferentiated stem cell-removing agent is composed of at least one kind selected from the group consisting of the fatty acid synthesis inhibitor, the fatty acid utilization inhibitor, and the cholesterol synthesis inhibitor, and may contain other optional components as long as the ability to remove undifferentiated stem cells is included.

The other components are not particularly limited, and examples thereof include pharmaceutically acceptable carriers, transfection accelerators, buffers, excipients, stabilizers, antioxidants, osmotic pressure regulators, pH adjusters, chelating agents, and the like.

A form of the undifferentiated stem cell-removing agent of the present embodiment is not particularly limited, and the agent may be in various forms such as a liquid matter, a powdery substance, a granular substance, a gel-like substance, and a solid matter. In addition, the agent may also be in an emulsion form which the fatty acid synthesis inhibitor, the fatty acid utilization inhibitor, the cholesterol synthesis inhibitor, or a combination thereof is encapsulated in micelles; or in a liposome form in which the fatty acid synthesis inhibitor, the fatty acid utilization inhibitor, the cholesterol synthesis inhibitor, or a combination thereof is encapsulated in liposomes.

Administration to a patient can be carried out parenterally or orally by methods known to those skilled in the art, for example. Parenteral administration methods include intranasal administration, transbronchial administration, intramuscular administration, transdermal administration, and the like, in addition to intraarterial injection, intravenous injection, subcutaneous injection, and the like. A dosage varies depending on a body weight and age of a patient, an administration method, and the like, but those skilled in the art can appropriately select an appropriate dosage.

For example, in a patient who has undergone transplantation of a differentiated cell tissue induced from an undifferentiated stem cell, it is possible to perform in vivo removal of undifferentiated stem cells remaining in the differentiated cell tissue by administration of the undifferentiated stem cell-removing agent of the present embodiment. Accordingly, diseases caused by proliferation of undifferentiated stem cells, such as canceration of transplanted tissue can be prevented or treated.

The undifferentiated stem cell-removing agent of the present embodiment can be used in a culture medium, a method for removing undifferentiated stem cells, a method for producing cells for transplantation, a pharmaceutical composition for treating or preventing diseases caused by proliferation of undifferentiated stem cells, a method for treating or preventing diseases caused by proliferation of undifferentiated stem cells, and the like, which will be described later.

An undifferentiated stem cell to be removed by the undifferentiated stem cell-removing agent of the present embodiment is not particularly limited, and is preferably iPS cell or ES cell and more preferably iPS cell.

### <<Culture Medium>>

In one embodiment, the present invention provides a culture medium. The culture medium of the present embodiment includes the undifferentiated stem cell-removing agent of the above embodiment. The culture medium of the present embodiment can be used for culturing cells. In the present specification, the term "culture" means breeding or growing cells outside a living body (individual), and includes so-called handling cells in vitro. The term "culture medium" refers to a liquid or solid substance that provides the culture environment to the cell.

The culture medium of the present embodiment may be a composition that contains, for example, an optional culture medium component and at least one kind selected from the group consisting of the fatty acid synthesis inhibitor, the fatty acid utilization inhibitor, and the cholesterol synthesis inhibitor (hereinafter referred to as the "fatty acid synthesis inhibitor and the like"). The culture medium of the present embodiment is a composition containing a medium and the fatty acid synthesis inhibitor and the like. Examples of the medium include water, a buffer solution, and the like. The medium may dissolve or disperse the fatty acid synthesis inhibitor and the like. The culture medium component may contain a component effective for cell growth, and examples of such components include various components such as amino acids, vitamins, inorganic salts, saccharides, and growth factors. In addition, components such as antibiotics, buffer solutions, chelating agents, and phenol red indicators may be contained.

The remaining components obtainable by removing the fatty acid synthesis inhibitor and the like from the culture medium of the present embodiment may have the same composition as a general cell culture solution used as a culture medium of the related art [for example, Dulbecco's Modified Eagle's Medium culture solution (DMEM), MEM culture solution (for example, α-MEM, MEM [Hank's BSS]), RPMI culture solution (for example, RPMI 1640 and the like), F12 culture solution, StemPro34, mTeSR1, and the like]. The above components may have the same composition as a general cell culture solution used as an undifferentiated-stem-cell maintenance culture medium [for example, StemFit medium, mTeSR (registered trademark) Essential 8 (registered trademark) medium, StemSure (registered trademark) medium, and the like].

A concentration of the fatty acid synthesis inhibitor and the like in the culture medium of the present embodiment may be the same as a concentration of the fatty acid synthesis inhibitor and the like in the application subject of the aforementioned undifferentiated stem cell-removing agent. With the culture medium of the present embodiment which contains the fatty acid synthesis inhibitor and the like at the above concentration, undifferentiated stem cells can be effectively removed. In addition, in a case where undifferentiated stem cells and differentiated cells coexist, undifferentiated stem cells can be removed while keeping the growth of differentiated cells in a favorable state.

The culture medium of the present embodiment preferably does not contain a fatty acid. In addition, the culture medium preferably does not contain a precursor of fatty acid synthesis (refer to FIG. 1) located downstream of a target of the fatty acid synthesis inhibitor used, so that fatty acids are not biosynthesized. Furthermore, the culture medium preferably does not contain substances located downstream of the target of the fatty acid utilization inhibitor used, so that fatty acids are not used. In addition, the culture medium of the present embodiment preferably does not contain cholesterol. In addition, the culture medium preferably does not contain a precursor of cholesterol synthesis located downstream of a target of the cholesterol synthesis inhibitor used, so that cholesterol is not biosynthesized.

The culture medium of the present embodiment may contain at least one compound selected from the group consisting of glucose, glutamine, and methionine. By culturing undifferentiated stem cells in a culture medium not containing these compounds, it is perceived that cell death of undifferentiated stem cells can be induced.

However, since the culture medium of the present embodiment contains the fatty acid synthesis inhibitor and the like, even if the culture medium contains at least one compound selected from the group consisting of glucose, glutamine, and methionine, the ability to remove undifferentiated stem cells is favorably exhibited. In addition, because the culture medium may contain glucose, glutamine, and methionine, it is expected that the growth of the cells becomes more favorable than a case where these components are not contained.

In addition, in another aspect, the present invention provides use of at least one kind selected from the group consisting of the fatty acid synthesis inhibitor, the fatty acid utilization inhibitor, and the cholesterol synthesis inhibitor, in the production of an undifferentiated stem cell-removing agent.

### <<Kit>>

In one embodiment, the present invention provides a kit including the undifferentiated stem cell-removing agent of the above embodiment.

In addition to the undifferentiated stem cell-removing agent of the above embodiment, the kit of the present embodiment further includes reagents, media, a cell culture instrument, instruction manual, and the like for inducing differentiated cells from undifferentiated stem cells. The reagents for inducing differentiated cells can be appropriately selected depending on differentiated cells to be induced. Examples of reagents for inducing cardiomyocytes include, but are not limited to, chromosomal DNA demethylating agents such as demethylase, 5-azacytidine, and DMSO; cytokines such as PDGF, fibroblast growth factor 8 (FGF-8), endothelin 1 (ET1), midkine and osteogenic factor 4 (BMP-4), and G-CSF; adhesive molecules such as gelatin, laminin, collagen, and fibronectin; vitamins such as retinoic acid; transcription factors such as Nkx2.5/Csx, GATA4, MEF-2A, MEF-2B, MEF-2C, MEF-2D, dHAND, eHAND, TEF-1, TEF-3, TEF-5, and MesP1; an extracellular matrix derived from cardiomyocytes; BMP antagonists such as noggin, chordin, fetuin, follistatin, sclerostin, Dan, Cerberus, gremlin, and dante; and the like. In addition, examples of the media include, but are not limited to, Dulbecco's Modified Eagle's Medium culture solution (DMEM), MEM culture solution (a-MEM, MEM [Hank's Bss], and the like), RPMI culture solution (RPMI 1640 and the like), F12 culture solution, StemPro 34, MTeSRI, StemFit medium, mTeSR Essential 8 medium, StemSure medium, and the like. Furthermore, examples of the cell culture instrument and the like include a cell culture plate and the like, but are not limited thereto.

The kit of the present embodiment can be suitably used for the method for removing undifferentiated stem cells, which is to be described later, and can further include instructions explaining the method for removing undifferentiated stem cells, and the like. The method for removing undifferentiated stem cells can be easily carried out in a short time by kitting reagents, instructions, and the like used for the method for removing undifferentiated stem cells, together with the undifferentiated stem cell-removing agent of the above embodiment.

### <<Method for Removing Undifferentiated Stem Cells>>

In one embodiment, the present invention provides a method for removing undifferentiated stem cells, which includes culturing a cell mixture that contains an undifferentiated stem cell and a differentiated cell in the presence of the undifferentiated stem cell-removing agent.

The undifferentiated stem cell-removing agent used in the method for removing undifferentiated stem cells of the present embodiment is the undifferentiated stem cell-removing agent of the above embodiment.

In the present specification, the term "cell mixture" refers to a cell population including two or more kinds of cells. The "cell mixture" may include components of the culture medium and the like, in addition to two or more kinds of cells. The "cell mixture containing undifferentiated stem cells and differentiated cells" includes one or more undifferentiated stem cells and one or more differentiated cells and may optionally include components of the culture medium and the like. A form of the cell mixture containing the undifferentiated stem cell and the differentiated cell is not particularly limited, and may be in an aggregated state, a dispersed state, a state of being adhered to a culture container, a state of being adhered to an adhesion factor such as an extracellular matrix, a sheet state, a lumpy state, a colony form, an embryoid body form, a cell clump form, a tissue form, an organ form, and the like.

When undifferentiated stem cells are subjected to a differentiation and induction procedure, differentiated cells are induced from undifferentiated stem cells. However, in in vitro, it is difficult to induce all undifferentiated stem cells into differentiated cells, and usually, undifferentiated stem cells remain, and thereby forming a cell mixture of undifferentiated stem cells and differentiated cells. In the method of the present embodiment, only undifferentiated stem cells can be selectively removed from such a cell mixture of undifferentiated stem cells and differentiated cells. In the method of the present embodiment, the cell mixture containing undifferentiated stem cells and differentiated cells may be a mixture obtainable by mixing undifferentiated stem cells and differentiated cells.

In the method of the present embodiment, an undifferentiated stem cell is not particularly limited, and is preferably iPS cell or ES cell and more preferably iPS cell.

In the method of the present embodiment, a differentiated cell is not particularly limited, and is preferably a cell differentiated and induced from undifferentiated stem cells of the same kind as the undifferentiated stem cells that coexist. Several methods for differentiating and inducing undifferentiated stem cells to differentiated cells have been reported so far, and differentiated cells can be induced using these known methods.

As the efficiency with which undifferentiated stem cells in the cell mixture can selectively be removed becomes higher, it becomes more preferable. In a case where a cell mixture is obtained by culturing the cell mixture containing undifferentiated stem cells and differentiated cells in the presence of the undifferentiated stem cell-removing agent of the above embodiment, a residual ratio of undifferentiated stem cells represented by the number of undifferentiated stem cells/(the number of undifferentiated stem cells + the number of differentiated cells) × 100 is preferably less than 0.1%, more preferably less than 0.01%, and even more preferably less than 0.001%. Dead cells are not included in the number of cells described above.

Undifferentiated stem cells remaining in the cell mixture may be detected by determining that cells expressing various markers specific to the undifferentiated stem cells are undifferentiated stem cells. Provided that, the above-mentioned residual ratio of undifferentiated stem cells is a ratio obtainable by determining that cells expressing Oct3/4 are undifferentiated stem cells, and by detecting undifferentiated stem cells remaining in the cell mixture. Here, it is sufficient that cells showing an expression level equivalent to an expression level of Oct3/4 in undifferentiated stem cells of the positive control are detected as cells expressing Oct3/4 by using undifferentiated stem cells cultured in a culture medium not containing the undifferentiated stem cell-removing agent of the above embodiment as a positive control.

Examples of differentiated cells may include cardiomyocytes, muscle cells, fibroblasts, nerve cells, immune cells (for example, lymphocytes and the like), vascular cells, ocular cells (for example, retinal pigment epithelial cells and the like), blood cells (for example, megakaryocytes, red blood cells, and the like), other tissue cells, progenitor cells thereof, and the like. Among them, cardiomyocytes or fibroblasts are preferred, and cardiomyocytes are more preferred.

In a case where cardiomyocytes are induced from pluripotent stem cells, it is perceived that the cells differentiate into cardiomyocytes via undifferentiated mesoderm, cardiac mesoderm (or predeterminate cardiomyocyte) as differentiation into cardiomyocytes progresses. Here, undifferentiated mesoderm refers to a stage in which expression of Brachyury protein specific to undifferentiated mesoderm is recognized. Meanwhile, cardiac mesoderm (or predeterminate cardiomyocytes) means cells in which expression of a protein specific to undifferentiated mesoderm, such as Brachyury, is observed, but expression of a protein specific to cardiomyocytes, such as Nkx2.5 and actinin is not observed in the same cell, and that has the ability to differentiate exclusively into cardiomyocytes without requiring the addition of a new substance to a culture solution. Cardiomyocyte means a cell performing autonomous pulsation. Cardiomyocytes express markers such as Nkx2.5, GATA4, and actinin. In the present specification, the term "cardiomyocyte" is used as a concept encompassing cardiomyocytes and cardiac mesoderm (or predeterminate cardiomyocyte).

Differentiation and induction from undifferentiated stem cells into cardiomyocytes can be carried out by using methods described in, for example, PCT International Publication No. WO01/048151, PCT International Publication No. WO2005/033298, PCT International Publication No. WO2008/150030, and the like. For example, differentiation and induction into cardiomyocytes may be performed by adding a substance that gives rise to differentiation into cardiomyocytes into a culture medium for culturing undifferentiated stem cells.

Examples of such substances include chromosomal DNA demethylating agents such as demethylase, 5-azacytidine, and DMSO; cytokines such as PDGF, fibroblast growth factor 8 (FGF-8), endothelin 1 (ET1), midkine and osteogenic factor 4 (BMP-4), and G-CSF; adhesive molecules such as gelatin, laminin, collagen, and fibronectin; vitamins such as retinoic acid; transcription factors such as Nkx2.5/Csx, GATA4, MEF-2A, MEF-2B, MEF-2C, MEF-2D, dHAND, eHAND, TEF-1, TEF-3, TEF-5, and MesP1; an extracellular matrix derived from cardiomyocytes; BMP antagonists such as noggin, chordin, fetuin, follistatin, sclerostin, Dan, Cerberus, gremlin, and dante; and the like.

Fibroblast means a cell having a fiber-producing ability. It may be determined whether the cells are fibroblasts based on properties specific to fibroblasts, such as fiber-producing ability and from expression of various markers. As a fibroblast marker, markers such as vimentin and ER-TR7 are known, and a cell positive for a marker can be determined to be a fibroblast.

The method of the present embodiment includes a step of culturing the cell mixture containing undifferentiated stem cells and differentiated cells in the presence of the undifferentiated stem cell-removing agent. This step can be carried out by adding the undifferentiated stem cell-removing agent into the culture medium for cell culture as described above, and culturing the cell mixture. Alternatively, the undifferentiated stem cell-removing agent may be added to a culture medium in which the cell mixture containing undifferentiated stem cells and differentiated cells is cultured.

An amount of the undifferentiated stem cell-removing agent added to the culture medium is not particularly limited, but is, for example, 0.1 to 500 µM, 1 to 50 µM, 3 to 15 µM, and the like as a final concentration when the agent is added to the culture medium.

Culturing of the cell mixture in the presence of the undifferentiated stem cell-removing agent may be carried out at a temperature generally used for cell culture. For example, a culturing temperature may be 20°C to 40°C, preferably 25°C to 38°C, and more preferably 30°C to 37°C.

A culturing time of the cell mixture in the presence of the undifferentiated stem cell-removing agent is not particularly limited, and is preferably 24 hours or more and more preferably 48 hours or more. The cells may be subcultured if necessary. Before and after subculturing, a composition of the culture medium may be the same as or different from each other as long as it contains the undifferentiated stem cell-removing agent.

A cell density of the cell mixture in the presence of the undifferentiated stem cell-removing agent is not particularly limited, and is preferably 1×10 to 1×10⁷ cells/mL, more preferably 1×10³ to 1×10⁶ cells/mL, and even more preferably 1×10⁴ to 1×10⁶ cells/mL.

According to the method of the present embodiment, the cell mixture from which undifferentiated stem cells have been removed has a reduced proportion of undifferentiated stem cells and is composed exclusively of differentiated cells. For this reason, according to the method for removing undifferentiated stem cells of the present embodiment, it is possible to obtain a cell mixture in which undifferentiated stem cells are substantially not contained, or a proportion of undifferentiated stem cells are reduced. Therefore, the cell mixture obtained by the method of the present embodiment can be suitably used as cells for transplantation into a living body.

In addition, in still another aspect, the present invention provides use of at least one kind selected from the group consisting of the fatty acid synthesis inhibitor, the fatty acid utilization inhibitor, and the cholesterol synthesis inhibitor, for removing undifferentiated stem cells.

### <<Method for Producing Cells for Transplantation>>

In one embodiment, the present invention provides a method for producing cells for transplantation including the following steps (i) and (ii):
a step (i) of inducing a desired differentiated cell from an undifferentiated stem cell; and
a step (ii) of culturing the cell mixture obtained in the step (i) in the presence of the undifferentiated stem cell-removing agent.

The undifferentiated stem cell-removing agent used in the method for producing cells for transplantation of the present embodiment is the undifferentiated stem cell-removing agent of the above embodiment.

The step (i) in the method of the present embodiment refers to a step of inducing a desired differentiated cell from an undifferentiated stem cell. An undifferentiated stem cell in the step (i) is not particularly limited, and is preferably iPS cell or ES cell and more preferably iPS cell.

In the step (i), the type of differentiated cells induced from undifferentiated stem cells is not particularly limited as long as differentiated cells are induced into desired differentiated cells. As a method for inducing an undifferentiated stem cell into a differentiated cell, a known method can be appropriately selected and used according a target differentiated cell. Examples of differentiated cells induced from undifferentiated stem cells include, but are not limited to, cardiomyocytes, muscle cells, fibroblasts, nerve cells, immune cells (for example, lymphocytes and the like), vascular cells, ocular cells (for example, retinal pigment epithelial cells and the like), blood cells (for example, megakaryocytes, red blood cells, and the like), other tissue cells, progenitor cells thereof, and the like. Examples of suitable differentiated cells include cardiomyocytes. The induction from undifferentiated stem cells into cardiomyocytes can be carried out by the method exemplified in aforementioned <<Method for Removing Undifferentiated Stem Cells>>.

Generally, in in vitro, because it is difficult to induce all undifferentiated stem cells into differentiated cells, undifferentiated stem cells usually remain in the cell mixture obtained in the step (i), and thereby forming a cell mixture of undifferentiated stem cells and differentiated cells. In addition, the cell mixture may optionally contain components of the culture medium and the like. A form of the cell mixture is not particularly limited, and may be in an aggregated state, a dispersed state, a state of being adhered to a culture container, a state of being adhered to an adhesion factor such as an extracellular matrix, a sheet state, a lumpy state, a colony form, an embryoid body form, a cell clump form, a tissue form, an organ form, and the like.

The step (ii) in the production method of the present embodiment refers to a step of culturing the cell population obtained in the step (i) in the presence of the undifferentiated stem cell-removing agent. The culture in this step (ii) can be carried out by the method exemplified in aforementioned <<Method for Removing Undifferentiated Stem Cells>>.

In the production method of the present embodiment, other steps may be added in addition to the above steps (i) and (ii). Examples of the other steps include a step of purifying differentiated cells, a step of collecting differentiated cells, and the like. In a case where these steps are added, these steps are carried out between the steps (i) and (ii) or after the step (ii).

In the step of purifying and the step of collecting differentiated cells, an appropriate method can be selected according to the type of differentiated cells. For example, in a case where a differentiated cell is a cardiomyocyte, methods described in PCT International Publication No. WO2006/022377, PCT International Publication No. WO2007/088874, PCT International Publication No. WO2016/010165, and the like may be applied to the purification step. In addition, as the collection step, a centrifugal separation method or the like may be applied.

In the cells for transplantation obtained by the production method of the present embodiment, a differentiated cell ratio represented by differentiated cell/(undifferentiated stem cell + differentiated cell) × 100 may be, for example, 50% or more, 70% or more, 80% or more, 90% or more, or 95% or more. Dead cells are not included in the number of cells described above.

In the cells for transplantation produced by the production method of the present embodiment, since undifferentiated stem cells have been selectively removed, a proportion of undifferentiated stem cells are reduced. Therefore the cells for transplantation are exclusively composed of differentiated cells. For this reason, according to the production method of the present embodiment, it is possible to obtain cells for transplantation, in which undifferentiated stem cells are substantially not contained, or a proportion of undifferentiated stem cells are reduced. Therefore, even in a case where the cells for transplantation are transplanted into a living body, the risk of teratoma formation and the like is reduced.

In addition, in still another aspect, the present invention provides use of at least one kind selected from the group consisting of the fatty acid synthesis inhibitor, the fatty acid utilization inhibitor, and the cholesterol synthesis inhibitor, for producing a cell for transplantation.

Furthermore, in still another aspect, the present invention provides cells for transplantation, produced by the method for producing cells for transplantation, the method including the following steps (i) and (ii):
a step (i) of inducing a desired differentiated cell from an undifferentiated stem cell; and
a step (ii) of culturing the cell mixture obtained in the step (i) in the presence of the undifferentiated stem cell-removing agent.

### <<Pharmaceutical composition>>

In one embodiment, the present invention provides a pharmaceutical composition for treating or preventing a disease caused by proliferation of an undifferentiated stem cell, in a subject into which a differentiated cell induced from the undifferentiated stem cell is transplanted, the pharmaceutical composition including the undifferentiated stem cell-removing agent.

The undifferentiated stem cell-removing agent contained in the pharmaceutical composition of the present embodiment is the same as that described in the above-mentioned <<Undifferentiated Stem Cell-Removing Agent>>.

A subject into which differentiated cells induced from undifferentiated stem cells are transplanted is not particularly limited, but may be a human or a non-human animal requiring transplantation of the differentiated cells. For example, the subject may be a patient having the differentiated cell not functioning normally, or a patient having a deficiency, a disorder, or the like in a tissue containing the differentiated cell. The non-human animal includes, but is not limited to, mammals. Examples of the mammals include primates such as monkeys; rodents such as mice and rats; pet animals such as dogs and cats; domestic animals such as cows, horses, sheep, and pigs; and the like. The subject into which differentiated cell induced from the undifferentiated stem cell is transplanted is preferably the same species as the organism from which the undifferentiated stem cell is derived, and more preferably the same individual as the individual from which the undifferentiated stem cell is derived. The subject into which differentiated cells induced from undifferentiated stem cells are to be transplanted may be a "subject requiring transplantation" described in <<Treatment Methods and the like>> to be described later.

A content of the fatty acid synthesis inhibitor and the like in the pharmaceutical composition of the present embodiment is not particularly limited so long as it is a therapeutically effective amount, and the content described in the above-mentioned <<Undifferentiated Stem Cell-Removing Agent>>, and the like can be exemplified.

The pharmaceutical composition of the present embodiment may contain other components in addition to the undifferentiated stem cell-removing agent. The other components are not particularly limited, and the components described in the above-mentioned <<Undifferentiated Stem Cell-Removing Agent>>, and the like can be exemplified.

In addition, the pharmaceutical composition of the present embodiment may contain an immunostimulating agent such as an adjuvant in addition to the other components described in the above-mentioned <<Undifferentiated Stem Cell-Removing Agent>>. Examples of the adjuvant include, but are not limited to, aluminum phosphate, aluminum hydroxide, alum, cholera toxin, Salmonella toxin, incomplete Freund's adjuvant (IFA), complete Freund's adjuvant (CFA) ISCOMATRIX, GM- CSF, CpG, O/W emulsion, and the like. The pharmaceutical composition of the present embodiment may contain other medicines having pharmacological activity, in addition to the undifferentiated stem cell-removing agent. Examples of the other medicines include anti-inflammatory agents, analgesic agents, antipyretic agents, compounds capable of immunity induction against undifferentiated stem cells, and the like.

A dosage form of the pharmaceutical composition of the present embodiment is not particularly limited and may be various dosage forms such as a liquid agent, a powder agent, a granule, a tablet, a powder, a suspension, an emulsion, an emulsion preparation, and a liposome preparation.

The pharmaceutical composition of the present embodiment is used for treating or preventing a disease caused by proliferation of an undifferentiated stem cell, in a subject into which a differentiated cell induced from the undifferentiated stem cell is transplanted. In general, because it is difficult to induce all undifferentiated stem cells into differentiated cells in vitro, undifferentiated stem cells may remain in differentiated cells prepared as cells for transplantation in some cases. In a case where undifferentiated stem cells remain transplanted, undifferentiated stem cells proliferate in a living body and may cause diseases such as teratoma. In order to treat or prevent such a disease, the pharmaceutical composition of the present embodiment is administered to a subject into which the differentiated cells are transplanted. Diseases caused by proliferation of undifferentiated stem cells are not particularly limited, and examples thereof include teratoma, cancer, and the like.

A differentiated cell to be transplanted into an administration subject of the pharmaceutical composition of the present embodiment is not particularly limited as long as it is a differentiated cell induced from an undifferentiated stem cell. Differentiated cells are preferably induced from iPS cells or ES cells, and more preferably induced from iPS cells. As an example, differentiated cells are cardiomyocytes induced from iPS cells. In addition, differentiated cells may be cells for transplantation produced by the method described in the aforementioned <<Method for Producing Cells for Transplantation>>.

An administration route of the pharmaceutical composition of the present embodiment can be appropriately selected depending on the type of active ingredients, a form of preparations, the type of differentiated cells transplanted, a place where differentiated cells are transplanted, and the like. For example, the administration route may be the same administration method as that described in the aforementioned <<Undifferentiated Stem Cell-Removing Agent>>.

A dosage and an administration interval of the pharmaceutical composition of the present embodiment can be appropriately selected according to the type of transplanted differentiated cells, an amount of transplantation, a place of transplantation, and the like; age, sex, body weight, and the like of a subject undergoing transplantation; an administration method of the pharmaceutical composition; and the like. A dosage may be, for example, 0.001 mg to 1000 mg, 0.01 mg to 100 mg, 0.1 mg to 30 mg, 0.1 mg to 10 mg, 0.5 mg to 5 mg, and the like. In addition, an administration interval may be one to several times per day, one time in several days to several months, and the like. For example, an administration in which an administration interval is once a day, once a week, or the like may be exemplified.

According to the pharmaceutical composition of the present embodiment, it is possible to perform in vivo selective removal of undifferentiated stem cells even in a case where undifferentiated stem cells remain in cells transplanted into a living body. Therefore, diseases caused by proliferation of undifferentiated stem cells in a living body can be treated or prevented.

In still another aspect, the present invention provides use of at least one kind selected from the group consisting of the fatty acid synthesis inhibitor, the fatty acid utilization inhibitor, and the cholesterol synthesis inhibitor, in the production of the pharmaceutical composition for treating or preventing a disease caused by proliferation of an undifferentiated stem cell, in a subject into which a differentiated cell induced from the undifferentiated stem cell is transplanted.

In addition, in still another aspect, the present invention provides use of at least one kind selected from the group consisting of the fatty acid synthesis inhibitor, the fatty acid utilization inhibitor, and the cholesterol synthesis inhibitor, for treating or preventing a disease caused by proliferation of an undifferentiated stem cell in a subject into which a differentiated cell induced from the undifferentiated stem cell is transplanted.

Furthermore, in still another aspect, the present invention provides at least one kind selected from the group consisting of the fatty acid synthesis inhibitor, the fatty acid utilization inhibitor, and the cholesterol synthesis inhibitor, for use in treating or preventing a disease caused by proliferation of an undifferentiated stem cell in a subject into which a differentiated cell induced from the undifferentiated stem cell is transplanted.

### <<Treatment Method and the like>>

In one embodiment, the present invention provides a method for treating or preventing a disease caused by proliferation of an undifferentiated stem cell, the method including administering the undifferentiated stem cell-removing agent to a subject into which a differentiated cell induced from the undifferentiated stem cell is transplanted.

The undifferentiated stem cell-removing agent to be administered to the subject in the method of the present embodiment is the same as that described in the above-mentioned <<Undifferentiated Stem Cell-Removing Agent>>. The method of the present embodiment is a method for treating or preventing a disease caused by proliferation of an undifferentiated stem cell, the method including administering at least one kind selected from the group consisting of the fatty acid synthesis inhibitor, the fatty acid utilization inhibitor, and the cholesterol synthesis inhibitor, to a subject into which a differentiated cell induced from the undifferentiated stem cell is transplanted.

In addition, the subject into which differentiated cells induced from undifferentiated stem cells are transplanted is the same as that described in the above-mentioned <<Pharmaceutical Composition>>. The undifferentiated stem cell-removing agent may be administered to the subject in the form of the pharmaceutical composition described in the above-mentioned <<Pharmaceutical Composition>>. In addition, the undifferentiated stem cell-removing agent may be combined with ingredients other than the fatty acid synthesis inhibitor and the like, so as to be administered to the subject. The other components are not particularly limited, and the components described in the above-mentioned <<Undifferentiated Stem Cell-Removing Agent>> and <<Pharmaceutical Composition>> can be exemplified.

A therapeutically effective amount of the fatty acid synthesis inhibitor and the like of the undifferentiated stem cell-removing agent is administered to the subject into which differentiated cells induced from undifferentiated stem cells is transplanted. A therapeutically effective amount of the fatty acid synthesis inhibitor and the like varies according to the type of transplanted differentiated cells, an amount of transplantation, a place of transplantation, and the like; age, sex, body weight, and the like of a subject undergoing transplantation; an administration method of the undifferentiated stem cell-removing agent; and the like. An active ingredient amount of the fatty acid synthesis inhibitor and the like may be, for example, 0.001 mg to 1000 mg, 0.01 mg to 100 mg, 0.1 mg to 30 mg, 0.1 mg to 10 mg, 0.5 mg to 5 mg, and the like.

An administration subject and a target disease can be the same as those described in the above-mentioned <<Pharmaceutical Composition>>. In addition, an administration method can be the same as that described in the above-mentioned <<Pharmaceutical Composition>>.

In addition, in one embodiment, the present invention provides a method for transplanting the cells for transplantation, the method including the following steps (i) to (iii):
a step (i) of inducing a desired differentiated cell from an undifferentiated stem cell;
a step (ii) of culturing the cell mixture obtained in the step (i) in the presence of the undifferentiated stem cell-removing agent so as to obtain the cells for transplantation; and
a step (iii) of transplanting the cells for transplantation obtained in the step (iii) to a subject requiring transplantation.

The steps (i) and (ii) in the transplantation method of the present embodiment can be carried out in the same manner as the steps (i) and (ii) described in the above-mentioned <<Method for Producing Cells for Transplantation>>. In addition, the undifferentiated stem cell-removing agent used in the step (ii) of the transplantation method of the present embodiment is the same as that described in the above-mentioned <<Undifferentiated Cell-Removing Agent>>.

The step (iii) in the transplantation method of the present embodiment is a step of transplanting the cells for transplantation obtained in the step (ii) to a subject requiring transplantation. The "subject requiring transplantation" is a subject in which defects, damages, or the like have occurred in a cell of the same type as the cells for transplantation obtained in the step (ii) in a living body of the object, and by transplanting the cells for transplantation, symptoms caused due to defects, damages, or the like of the cells are expected to be ameliorated. Transplantation can be carried out by a general method of cell transplantation.

In the transplantation method of the present embodiment, other steps may be added in addition to the above steps (i) to (iii). Examples of the other steps include a step of purifying differentiated cells, a step of collecting differentiated cells, and the like. In a case where these steps are added, these steps are carried out between the steps (ii) and (iii). Such a purification step and collection step can be carried out as described in <<Method for Producing Cells for Transplantation>>.

According to the transplantation method of the present embodiment, the cells for transplantation in which undifferentiated stem cells are substantially not contained, or a ratio of undifferentiated stem cells is reduced, can be transplanted into a living body. Therefore the risk of teratoma formation or the like is reduced.

In addition, in the transplantation method of the present embodiment, the following step (iv) may be further performed after the step (iii).

The step (iv) of administering the undifferentiated stem cell-removing agent to the subject into which the cells for transplantation are transplanted in the step (iii).

The above step (iv) can be carried out in the same manner as the treatment or prevention method of the above embodiment. By carrying out the step (iv), even in a case where undifferentiated stem cells remain transplanted into the cells for transplantation, it is possible to prevent the onset of diseases caused by proliferation of the undifferentiated stem cells, such as teratomas.

Hereinafter, the present invention will be described based on examples, but the present invention is not limited to the following examples.

### Examples

The cells were cultured in an incubator under conditions of 37°C and 5% CO₂.

### (Culture of Undifferentiated Stem Cells)

An H9 strain obtained from the WiCell Research Institute was used as human embryonic stem cells, and human induced pluripotent stem cells were obtained from a national university corporation · Professor Shinya Yamanaka at the iPS Cell Research Institute, Kyoto University.

The human embryonic stem cells and the human induced pluripotent stem cells were subjected to undifferentiated maintenance culture using Matrigel (BD Bioscience cat 354277). As a culture solution, mTeSR1 (STEMCELL Technologies Inc. cat 11875-119) was used. In addition to mTeSR1, any culture solution for undifferentiation maintenance may be used as long as it is generally used as a feeder-free culture medium such as Essential 8 (Life Technologies), TeSR2 (STEMCELL Technologies Inc.), and the like. In addition, matrices include Vitronectin (Life Technologies), iMatrix-511 (Takara no. 892001), and the like, in addition to Matrigel, and any matrix can be used as long as it is commonly used as other feeder-free matrix.

Upon subculturing, colonies of human embryonic stem cells and induced pluripotent stem cells were isolated by CTK solution (Repro CELL), at 37°C for 5 minutes. Regarding cell dispersion processing, StemPro Accutase (Life Technologies no. 1110501) and TrypLE Express/Select (Life Technologies) can also be used in addition to the CTK solution.

### (Induction of Cardiomyocytes from Undifferentiated Stem Cells)

In regard to the differentiation and induction from undifferentiated stem cells into cardiomyocytes, a method for differentiating and inducing cardiomyocytes used in this experiment is as follows.

• In regard to the differentiation and induction into cardiomyocytes, when human embryonic stem cells or human induced pluripotent stem cells became 50% to 90% confluent, a culture medium was changed to a culture medium in which 6 µM of B27 (no insulin, Invitrogen) and CHIR99021 (Selleckchemor Wako) were added into RPMI medium (Invitrogen) (Day 0).
- In Day 1 to Day 2, culturing in RPMI/B27 insulin(-) medium was carried out.
- Next, in Day 3 to Day 5, culturing in a culture medium into which 5 µM of IWP2 or 5 µM of IWR-1 (Sigma 10161) was added in addition to RPMI/B27 insulin(-) medium was carried out.
- Furthermore, in Day 6 to Day 7, culturing in RPMI/B27 insulin(-) medium was carried out.
- In addition, from Day 8 onwards, culturing was carried out in RPMI/B27 insulin(+) medium (Lian, X., et al., Nat Protocol, 2013, 8, 162-175). At the stage of Day 8 to Day 11, pulsating cardiomyocytes could be confirmed.

### (Induction of Fibroblasts from Undifferentiated Stem Cells)

In regard to the differentiation and induction from undifferentiated stem cells into fibroblasts, a method for inducing fibroblasts used in this experiment is as follows.
- When the human embryonic stem cells or human induced pluripotent stem cells became 50% to 90% confluent, a culture medium was exchanged to a culture solution (containing no bFGF) in which 10% FBS was added into MEMα medium (Invitrogen) so as to carry out the culture. The culture solution was changed every 2 to 4 days. Therefore Vimentin-positive fibroblasts could be obtained in about 10 days (refer to Tohyama S, Cell Metabolism 2016).

### [Experimental Example 1] Comparison of FASN Expression between Cardiomyocytes Derived from Human iPS Cells and iPS Cells

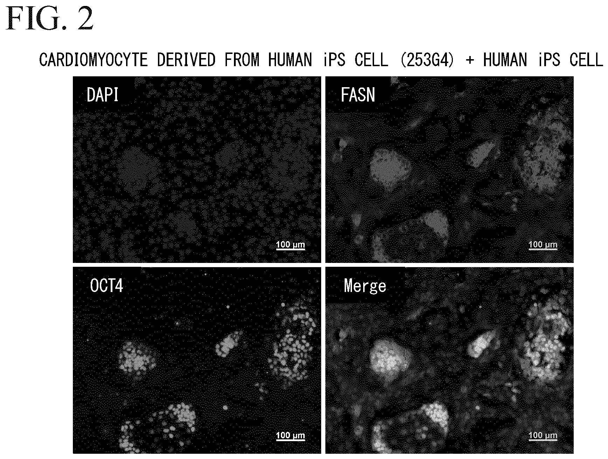

A cell mixture in a state in which cardiomyocytes induced from human iPS cells and human iPS cells coexisted was prepared according to the procedure described above. OCT4 was used as a marker representing an undifferentiated state. As shown in FIG. 1, FASN is an enzyme protein involved in fatty acid synthesis. Cells expressing OCT4 and FASN were detected by immunostaining of the cell mixture. The results are shown in FIG. 2. In addition, the results of DAPI staining are also shown together.

Based on the results shown in FIG. 2, it was found that the cells expressing OCT4 and FASN overlapped. Based on the above observation, it was elucidated that the expression of FASN was low in cardiomyocytes derived from human iPS cells which do not express OCT4, whereas the expression of FASN was very high in undifferentiated human iPS cells expressing OCT4.

### [Experimental Example 2] Culture of Various Undifferentiated Stem Cell Lines in Culture Medium Containing Fatty Acid Synthesis Inhibitor

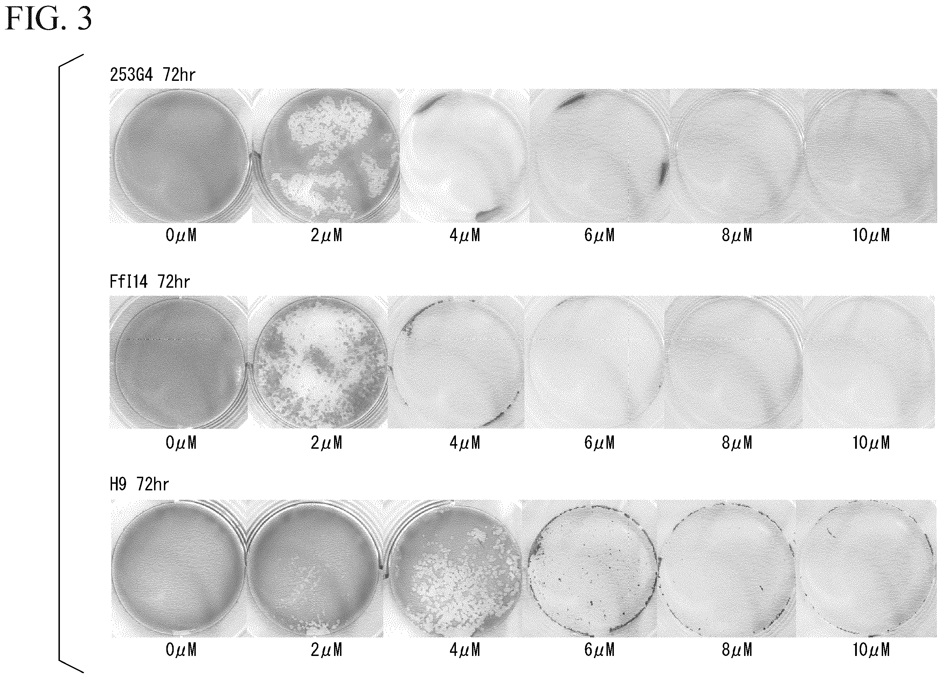

Orlistat (manufactured by Sigma, 04139) was added into StemFit medium (manufactured by AJINOMOTO) or mTeSR1 (manufactured by Stem Cell Technologies) so that each of a final concentration became concentrations as shown in FIG. 3 (2 µM, 4 µM, 6 µM, 8 µM, and 10 µM). Therefore a culture medium according to one embodiment of the present invention was obtained. The following three cell lines were cultured for 72 hours in each of a culture medium not containing Orlistat (0 µM) and the culture media containing Orlistat at each of the above final concentrations.
- 253G4: human induced pluripotent stem cell (human iPS cell) line
- Ffl14: human induced pluripotent stem cell (human iPS cell) line
- H9: human embryonic stem cell (human ES cell) line

In regard to the activity of alkaline phosphatase (ALP) that the cells have, after 72 hours of culture, AP staining was performed by color development with StemTAG (registered trademark) alkaline phosphatase staining kit (Sigma 86-R), and cells stained red were confirmed as living cells. The state of each well is shown in FIG. 3.

Based on the results shown in FIG. 3, it was confirmed that cell death of each undifferentiated stem cell was induced in an Orlistat concentration-dependent manner by culturing in the culture medium containing Orlistat.

### [Experimental Example 3] Culture of Undifferentiated Stem Cell Line in Culture Medium Containing Various Fatty Acid Synthesis Inhibitors or Fatty Acid Utilization Inhibitors

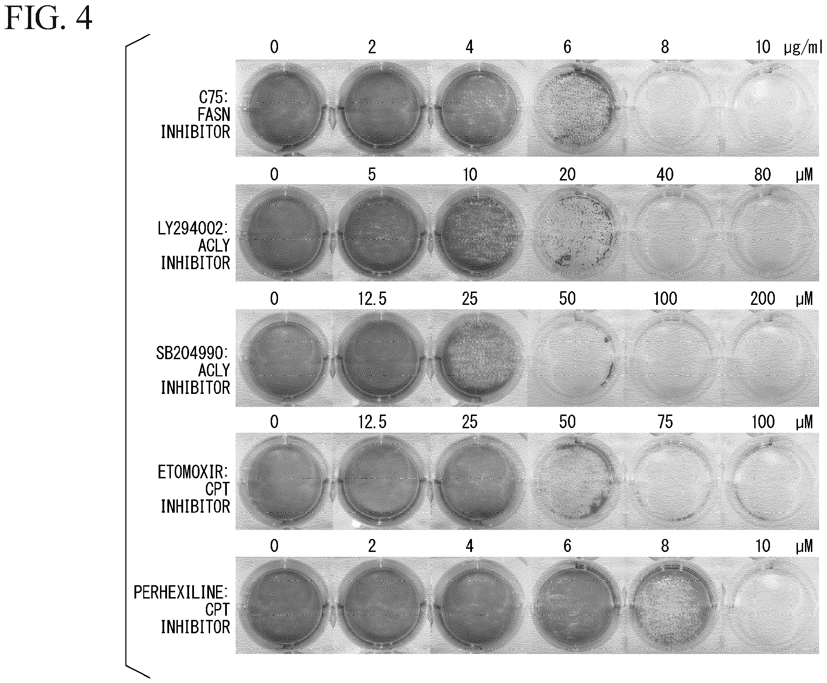

Each of six kinds of fatty acid synthesis inhibitors or fatty acid utilization inhibitors shown below were added into StemFit medium (manufactured by AJINOMOTO CO., LTD.) or mTeSR1 medium (manufactured by Stem Cell Technologies, Inc.) so that each of a final concentration became a concentration as shown in FIG. 4. Therefore a culture medium according to one embodiment of the present invention was obtained.
- C75: FASN inhibition
- LY 294002: ACLY inhibition
- SB 204990: ACLY inhibition
- Etomoxier: CPT1 inhibition
- Perhexiline: CPT1 inhibition

253G4 cells were cultured for 72 hours in each culture medium containing the above fatty acid synthesis inhibitor or fatty acid utilization inhibitor.

After 72 hours of culture, ALP staining was performed, and cells stained red were confirmed as living cells. The state of each well is shown in FIG. 4.

Based on the results shown in FIG. 4, it was confirmed that, when culturing in the culture medium containing any of the above-mentioned fatty acid synthesis inhibitors or fatty acid utilization inhibitors, cell death of undifferentiated stem cells was induced in a manner dependent on a concentration of the fatty acid synthesis inhibitor or fatty acid utilization inhibitor.

### [Experimental Example 4] Culture of Purified and Refined Cardiomyocytes in Culture Medium Containing Fatty Acid Synthesis Inhibitor

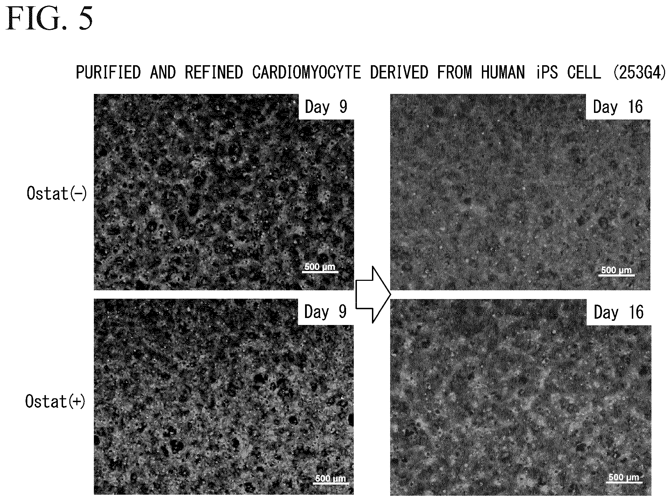

A cell mixture containing cardiomyocytes induced from human iPS cells and human iPS cells was prepared according to the procedure described above. Following the method described in the literature (Tohyama, et al., (2016) Cell Metabolism, 23, 663-674.), the above cell mixture was cultured for about 5 days in a glucose(-) glutamine(-) lactic acid-added culture medium so as to kill cells other than cardiomyocytes. Therefore purified and refined cardiomyocytes were obtained. Subsequently, culturing was carried out for 16 days in a MEMα + 10% FBS culture medium (Orlistat(+)) containing Orlistat at a final concentration of 10 µM. As a control, culturing was carried out in the same manner in a MEMα + 10% FBS culture medium (Orlistat(-)) not containing Orlistat. The state of cardiomyocytes on Day 9 and Day 16 is shown in FIG. 5.

Based on the results shown in FIG. 5, maturation of the cardiomyocytes (enlargement) was confirmed from Day 9 to Day 16, and it was shown that purified and refined cardiomyocytes can be favorably cultured using the culture medium according to the present embodiment.

### [Experimental Example 5] Culture of Fibroblasts in Culture Medium Containing Fatty Acid Synthesis Inhibitor

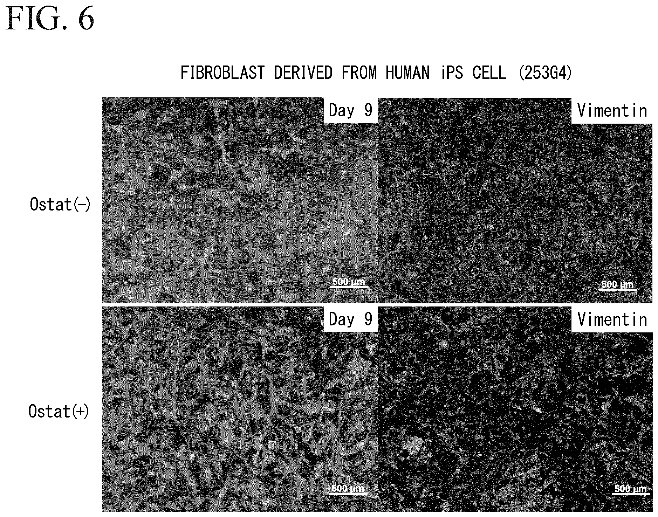

A cell mixture containing fibroblasts induced from human iPS cells and human iPS cells was prepared according to the procedure described above. Subsequently, the cell mixture was cultured for 9 days in a MEMα + 10% FBS culture medium (Orlistat(+)) containing Orlistat at a final concentration of 10 µM. As a control, the cell mixture was cultured in the same manner in a MEMα + 10% FBS culture medium (Orlistat(-)) not containing Orlistat. The cardiomyocytes on Day 9 and Vimentin staining results thereof are shown in FIG. 6.

Based on the results shown in FIG. 6, it was shown that the fibroblasts derived from human iPS cells can be favorably cultured using the culture medium according to one embodiment of the present invention.

### [Experimental Example 6] Culture of Cell Mixture Containing Cardiomyocytes Derived from Human iPS Cells and iPS Cells in Culture Medium Containing Fatty Acid Synthesis Inhibitor

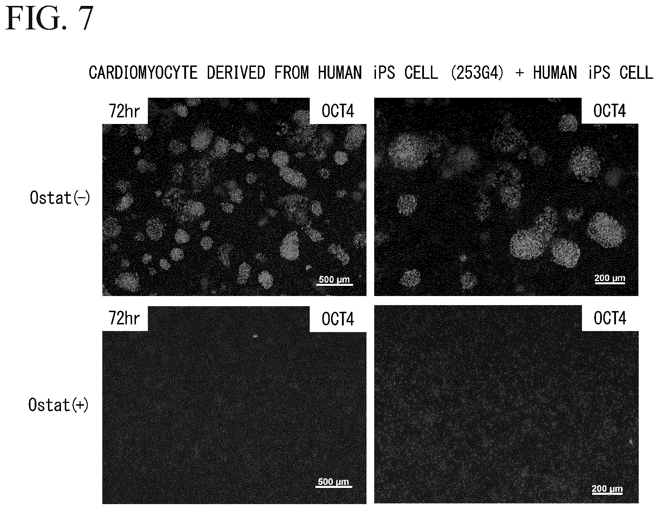

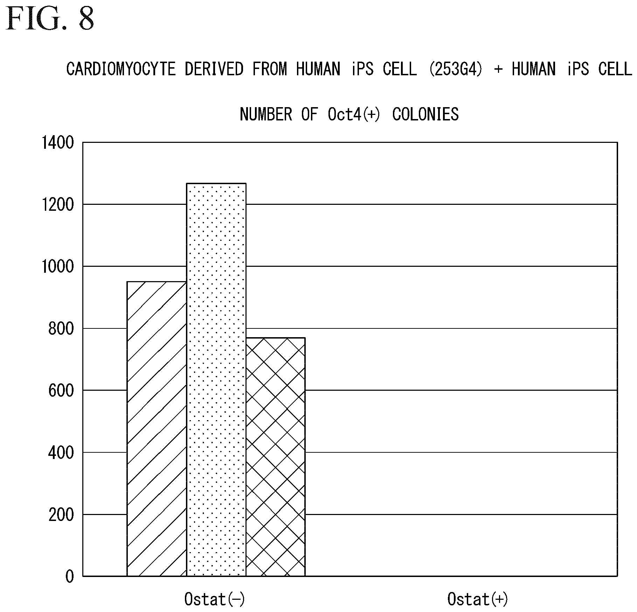

A cell mixture in a state in which purified and refined cardiomyocytes induced from human iPS cells and human iPS cells coexisted was prepared according to the procedure described above. In the cell mixture, immunostaining against OCT4 was performed. The cell mixture was cultured for 72 hours in StemFit medium (manufactured by AJINOMOTO) or mTeSRl (manufactured by Stem Cell Technologies) containing Orlistat at a final concentration of 10 µM (Orlistat(+)). As a control, culture was carried out in the same manner in StemFit medium (manufactured by AJINOMOTO) or mTeSR1 (manufactured by Stem Cell Technologies) not containing Orlistat (Orlistat(-)). After culturing for 72 hours, cells expressing OCT4 were detected by immunostaining. The results of two replicate tests are shown in FIG. 7. In addition, the results of DAPI staining are also shown together. The results of three replicate tests in which the number of colonies of cells expressing Oct4 was quantified based on the results are shown in FIG. 8.

Based on the results shown in FIGS. 7 to 8, it was confirmed that undifferentiated stem cells expressing OCT4 remained in a case where culturing was carried out in the culture medium not containing Orlistat (Orlistat(-)). On the other hand, in a case where culturing was carried out in the culture medium containing Orlistat (Orlistat(+)), no residual undifferentiated stem cells expressing OCT4 were detected.

Based on these findings, by culturing the cell mixture in a state in which cardiomyocytes derived from human iPS cells and human iPS cells coexist in the undifferentiated stem cell-removing agent according to one embodiment of the present invention, it is possible to induce cell death specific to cells in an undifferentiated state in the cell mixture. In addition, it was shown that only cells in a differentiated state that include cardiomyocytes derived from human iPS cells can be selectively selected from the cell mixture.

In Experimental Example 6, cells having the properties of undifferentiated stem cells were excluded from the cell mixture thus obtained. Therefore a removal rate of undifferentiated stem cells was also very high. The cell mixture is expected to be applied to applications such as transplantation into a living body, and shows outstanding and remarkable usefulness.

### [Experimental Example 7] Comparison of FASN Expression between Cardiomyocytes Derived from Human iPS Cells and iPS Cells

A cell mixture in a state in which cardiomyocytes induced from human iPS cells and human iPS cells coexisted was prepared according to the procedure described above. Troponin 1 (TnI) was used as a cardiomyocyte marker. As shown in FIG. 1, FASN is an enzyme protein involved in fatty acid synthesis. Cells expressing TnI and FASN were detected by immunostaining of the cell mixture. The results are shown in FIG. 10. In addition, the results of DAPI staining are also shown together.

Based on the results shown in FIG. 10, it was found that the cells expressing TnI and FASN did not overlap. Based on the above observation, it was elucidated that the expression of FASN was low in cardiomyocytes derived from human iPS cells which express TnI, whereas the expression of FASN was very high in undifferentiated human iPS cells which do not express TnI.

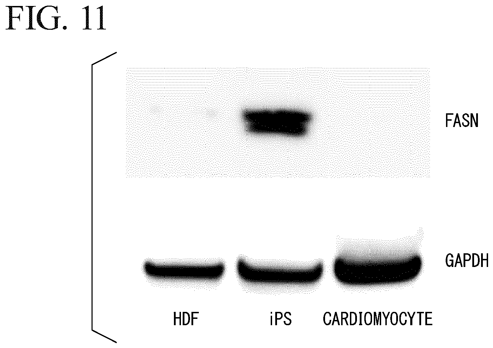

In addition, expression of FASN in cardiomyocytes induced from human iPS cells (253G4) and human iPS cells was confirmed by Western blotting using an anti-FASN antibody. As a negative control, human skin fibroblasts (HDF) were used. The results are shown in FIG. 11.

Based on the results shown in FIG. 11, it was shown that expression of FSAN was not detected in cardiomyocytes derived from human iPS cells, and FASN was highly expressed in human iPS cells.

### [Experimental Example 8] Culture of Undifferentiated Stem Cell Line in Culture Medium Containing Cholesterol Synthesis Inhibitor

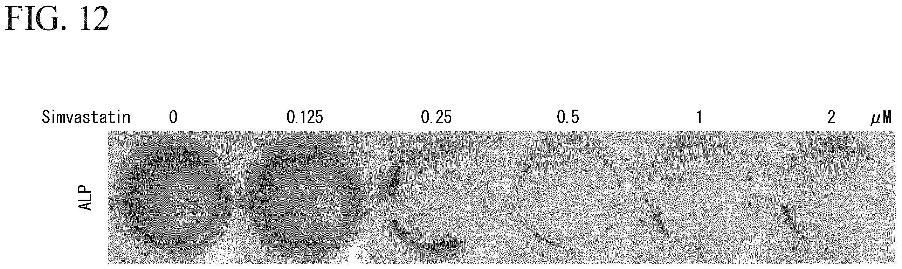

Simvastatin (S6196: manufactured by Sigma Aldrich) was added into StemFit medium (manufactured by AJINOMOTO) or mTeSRl medium (manufactured by Stem Cell Technologies) so that each of a final concentration became concentrations as shown in FIG. 12 (0.125 µM, 0.25 µM, 0.5 µM, 1 µM, and 2 µM). Therefore a culture medium according to one embodiment of the present invention was obtained.

253G4 cells were cultured for 72 hours in each of a culture medium not containing Simvastatin (0 µM) and the media containing Simvastatin at each of the above final concentrations.

After 72 hours of culture, ALP staining was performed, and cells stained red were confirmed as living cells. The state of each well is shown in FIG. 12.

Based on the results shown in FIG. 12, it was confirmed that cell death of each undifferentiated stem cell was also induced in a Simvastatin concentration-dependent manner by culturing in the culture medium containing Simvastatin.

### [Experimental Example 9] Culture of Undifferentiated Stem Cell Line in Medium culture Containing Fatty Acid Synthesis Inhibitor and Palmitic Acid

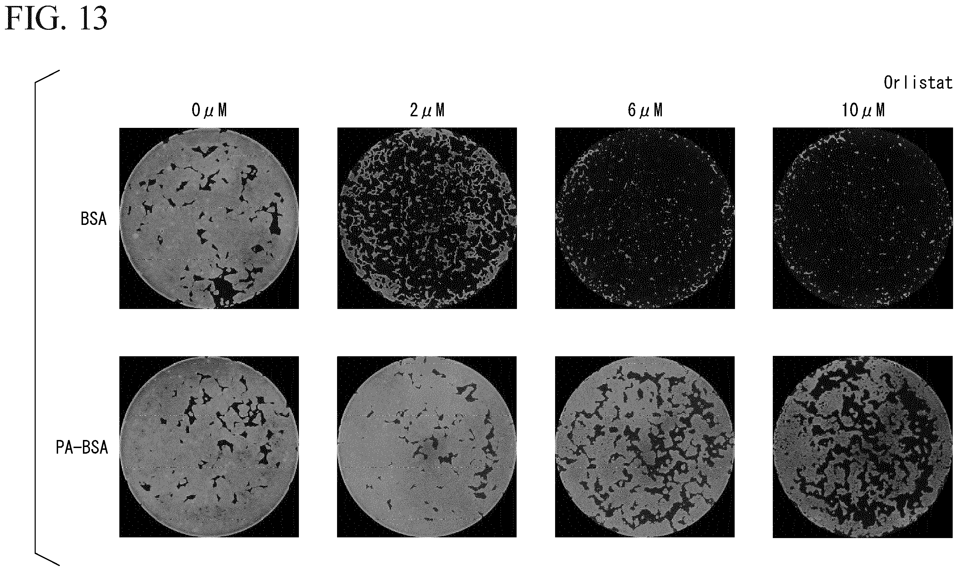

Orlistat (manufactured by Sigma, 04139) was added into StemFit medium (manufactured by AJINOMOTO) or mTeSR1 (manufactured by Stem Cell Technologies) so that each of a final concentration became concentrations as shown in FIG. 13 (2 µM, 6 µM, and 8 µM). Therefore a culture medium according to one embodiment of the present invention was obtained. Media obtained by adding palmitic acid (final concentration 50 µM), carnitine (final concentration 0.5 mM), and BSA (final concentration 8.3 µM) to each of the culture medium (0 µM) not containing Orlistat and the culture media containing Orlistat at the above final concentrations; and media obtained by adding only carnitine (final concentration 0.5 mM) and BSA (final concentration 8.3 µM) thereto were prepared. The human iPS cell line (253G4) was cultured for 72 hours in each of the above-mentioned media.

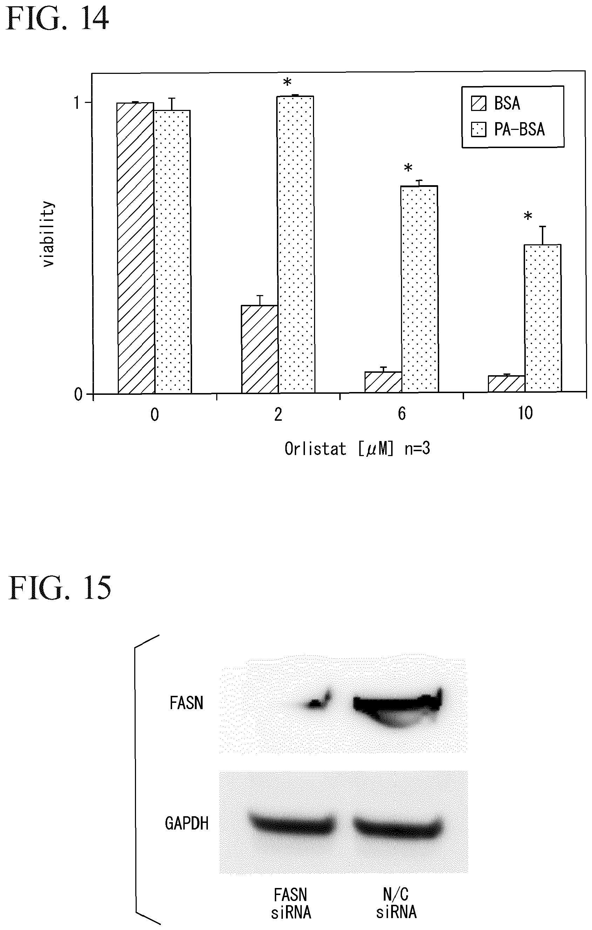

After 72 hours of culture, live cells were detected with LIVE/DEAD Assay (L3224, manufactured by Thermo Fisher Scientific). The state of each well is shown in FIG. 13. In addition, the result of quantifying the number of viable cells based on the same result is shown in FIG. 14.

Based on the results shown in FIGS. 13 to 14, it was confirmed that cell death of human iPS cells was induced in an Orlistat concentration-dependent manner by culturing in the culture medium (BSA) into which only carnitine and BSA were added in addition to Orlistat. On the other hand, it was confirmed that cell death of human iPS cells was suppressed by culturing in the culture medium (PA-BSA) into which palmitic acid, carnitine, and BSA were added in addition to Orlistat. Based on the finding that cell death of human iPS cells was suppressed by addition of palmitic acid, which is a fatty acid, it was confirmed that induction of cell death of human iPS cells by Orlistat was caused by inhibition of fatty acid synthesis.

### [Experimental Example 10] Culture of Undifferentiated Stem cell Line with Knock-Down of FASN

A FASN-knock-down undifferentiated stem cell line (FASN KD undifferentiated stem cell line) was produced by introducing siRNA (FASN siRNA) against FASN into a human iPS cell line (253G4). In addition, an undifferentiated stem cell line into which siRNA of negative control (N/C) was introduced was prepared as a negative control line (N/C undifferentiated stem cell line). The result of confirming FASN expression in FASN KD undifferentiated stem cell line and N/C undifferentiated stem cell line by Western blotting using an anti-FASN antibody is shown in FIG. 15. Based on the results in FIG. 15, it was confirmed that the expression of FASN could be knocked down by introduction of FASN siRNA. Each siRNA used was as follows.

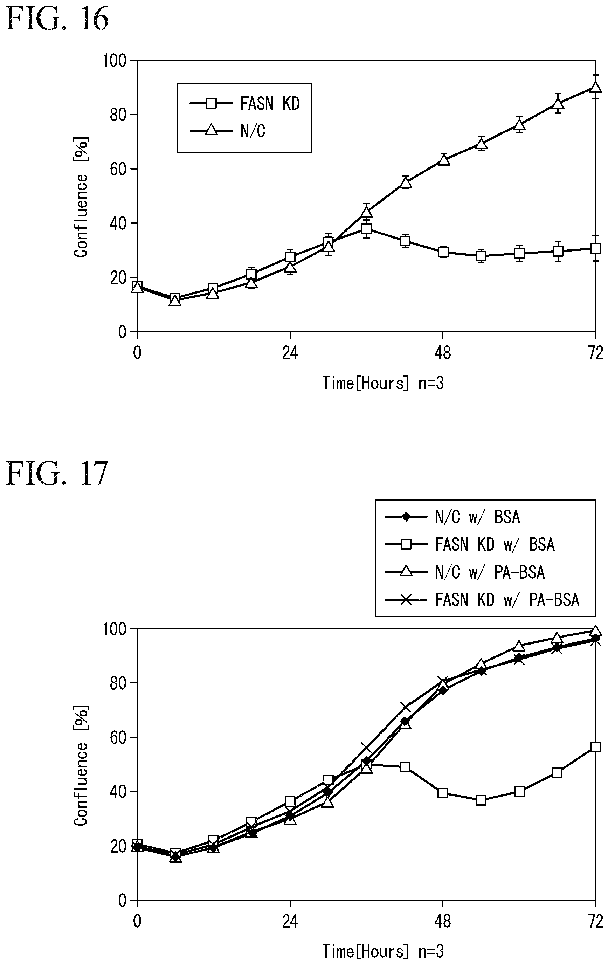

FASN siRNA: Applied Biosystems (registered trademark) siRNA ID: s5030 (manufactured by Thermo Fisher Scientific)
N/C siRNA: Silencer™ Negative Control No. 1 siRNA (AM 4611: manufactured by Thermo Fisher Scientific)
The above FASN KD undifferentiated stem cell line and N/C undifferentiated stem cell line were cultured for 72 hours in a StemFit medium (manufactured by AJINOMOTO) or mTeSRl (manufactured by Stem Cell Technologies) to confirm cell proliferation. The results are shown in FIG. 16.

Based on the results shown in FIG. 16, it was confirmed that cell proliferation was suppressed in the FASN KD undifferentiated stem cell line as compared with the N/C undifferentiated stem cell line. Based on this finding, it was shown that FSAN is an essential factor for the survival and proliferation of undifferentiated stem cells.

### [Experimental Example 11] Acid Culture of FASN KD Undifferentiated Stem Cell Line in Culture Medium Containing Palmitic Acid

Media (PA-BSA media) obtained by adding palmitic acid (final concentration 50 µM), carnitine (final concentration 0.5 mM), and BSA (final concentration 8.3 µM) to StemFit medium (manufactured by AJINOMOTO) or mTeSR1 (manufactured by Stem Cell Technologies); and media (BSA media) obtained by adding only carnitine (final concentration 0.5 mM) and BSA (final concentration 8.3 µM) thereto were prepared. The FASN KD undifferentiated cell line and the N/C undifferentiated cell line prepared in Experimental Example 11 were cultured in each of the above-mentioned culture medium for 72 hours to confirm cell proliferation. The results are shown in FIG. 17.

Based on the results shown in FIG. 17, the FASN KD undifferentiated stem cell line cultured in the PA-BSA culture medium (FASN KD w/PA-BSA), the N/C undifferentiated stem cell line cultured in the BSA-PA culture medium (N/C w/PA-BSA), and the N/C undifferentiated stem cell line cultured in the BSA culture medium (N/C w/BSA) showed the same level of cell proliferation. On the other hand, cell proliferation was suppressed in the FASN KD undifferentiated stem cell line cultured in the BSA culture medium, as compared with the above three lines. Based on these findings, it was shown that palmitic acid synthesized by FASN is an essential component for the survival and proliferation of undifferentiated stem cells.

### Industrial Applicability

According to the present invention, the undifferentiated stem cell-removing agent which is capable of removing undifferentiated stem cells with high efficiency, the method for removing undifferentiated stem cells, and the like can be provided and can be widely used in the field of regenerative medical techniques, and the like.

## Claims

1. An undifferentiated stem cell-removing agent, comprising:
at least one kind selected from the group consisting of a fatty acid synthesis inhibitor, a fatty acid utilization inhibitor, and a cholesterol synthesis inhibitor.

2. The undifferentiated stem cell-removing agent according to Claim 1,
wherein the fatty acid synthesis inhibitor inhibits fatty acid synthesis by targeting at least one factor selected from the group consisting of an ATP citrate lyase, a fatty acid synthase, an acetyl-CoA carboxylase, and a malonyl-CoA decarboxylase, and
the fatty acid utilization inhibitor inhibits fatty acid utilization by targeting a carnitine palmitoyltransferase 1.

3. The undifferentiated stem cell-removing agent according to Claim 1 or 2,
wherein a concentration of the fatty acid synthesis inhibitor or the fatty acid utilization inhibitor in an application subject is 0.1 to 500 µM.

4. The undifferentiated stem cell-removing agent according to Claim 1,
wherein the cholesterol synthesis inhibitor inhibits cholesterol synthesis by targeting at least one factor selected from the group consisting of an acetyl-CoA acetyltransferase, an HMG-CoA synthase, and an HMG-CoA reductase.

5. The undifferentiated stem cell-removing agent according to Claim 1 or 4,
wherein a concentration of the cholesterol synthesis inhibitor in an application subject is 0.01 to 50 µM.

6. The undifferentiated stem cell-removing agent according to any one of Claims 1 to 5, further comprising:
at least one compound selected from the group consisting of glucose, glutamine, and methionine.

7. An undifferentiated stem cell-removing agent, comprising:
one or two or more kinds selected from the group consisting of Orlistat, C75, LY294002, SB204990, etomoxir, perhexiline, and simvastatin, and salts thereof.

8. A culture medium, comprising:
the undifferentiated stem cell-removing agent according to any one of Claims 1 to 7.

9. A method for removing undifferentiated stem cells, comprising:
culturing a cell mixture that contains an undifferentiated stem cell and a differentiated cell in the presence of the undifferentiated stem cell-removing agent according to any one of Claims 1 to 7.

10. The method for removing undifferentiated stem cells according to Claim 9,
wherein the undifferentiated stem cell is an induced pluripotent stem cell.

11. The method for removing undifferentiated stem cells according to Claim 10 or 11,
wherein the differentiated cell is a cardiomyocyte.

12. A production method of cells for transplantation, comprising the following steps (i) and (ii):
a step (i) of inducing a desired differentiated cell from an undifferentiated stem cell; and
a step (ii) of culturing a cell mixture obtained in the step (i) in the presence of the undifferentiated stem cell-removing agent according to any one of Claims 1 to 7.

13. The production method according to Claim 12,
wherein the undifferentiated stem cell is an induced pluripotent stem cell.

14. The production method according to Claim 12 or 13,
wherein the differentiated cell is a cardiomyocyte.

15. A pharmaceutical composition for treating or preventing a disease caused by proliferation of an undifferentiated stem cell in a subject into which a differentiated cell induced from the undifferentiated stem cell is transplanted, the pharmaceutical composition comprising:
the undifferentiated stem cell-removing agent according to any one of Claims 1 to 7.

16. The pharmaceutical composition according to Claim 15,
wherein the undifferentiated stem cell is an induced pluripotent stem cell.

17. The pharmaceutical composition according to Claim 15 or 16,
wherein the differentiated cell induced from the undifferentiated stem cell is a cardiomyocyte.
